# NEW EUROPEAN PATENT SPECIFICATION

(11) **EP 0 135 587 B2**
(45) Date of publication and mention of the opposition decision: **18.12.2002**
(45) Mention of the grant of the patent: 02.05.1990
(21) Application number: 84901462.6
(22) Date of filing: 22.02.1984
(51) Int. Cl.: C07H 21/00, C12Q 1/68, C07H 17/00, C07H 19/06, C07H 15/12

(54) **DEFINED SEQUENCE SINGLE STRAND OLIGONUCLEOTIDES INCORPORATING REPORTER GROUPS, PROCESS FOR THE CHEMICAL SYNTHESIS THEREOF, AND NUCLEOSIDES USEFUL IN SUCH SYNTHESIS**
EINZELSTRANG-OLIGONUKLEOTIDE BESTIMMTER SEQUENZ, MARKERGRUPPEN ENTHALTEND, VERFAHREN ZU DEREN CHEMISCHER SYNTHESE UND NUKLEOTIDE ZUR VERWENDUNG DARIN
OLIGONUCLEOTIDES A BRIN UNIQUE ET A SEQUENCE DEFINIE COMPRENANT DES GROUPES RAPPORTEURS, PROCEDE POUR EFFECTUER LEUR SYNTHESE CHIMIQUE, ET NUCLEOSIDES UTILES DANS UNE TELLE SYNTHESE

(30) Priority: 22.02.1983 WO PCT/AU83/00254; 22.02.1983 US 468498
(43) Date of publication of application: 03.04.1985
(73) Proprietor: SYNGENE, INC., San Diego, CA 92121-2789 (US)
(72) Inventor: RUTH, Jerry, L., Carlsbad, CA 92008 (US)
(74) Representative: Dost, Wolfgang, Dr.rer.nat., Dipl.-Chem.
(86) International application number: US8400279
(87) International publication number: WO84003285

(56) References cited:
- EP-A- 0 063 879
- EP-A- 0 070 687
- WO-A-83/01459
- WO-A-83/02286
- WO-A-83/03260
- US-A- 4 199 498
- US-A- 4 247 544
- US-A- 4 267 171
- US-A- 4 336 188
- US-A- 4 358 535
- Reines et al., A New Method for the attachment of Flourescent Probes to tRNA, Chemical Abstracts 97:68868x (1981)
- Alvarado-Urbina et al., Automated Synthesis of Gene Fragments, Science 214, 270 (1981)
- Letsiger et al., Synthesis of Thymidine Oligonucleotides by Phosphite Triester Intermediates, J. Am. Chem. Soc. 98(12), 3655 (1976)
- Jayaraman et al., Solid-Phase 'Phosphite' Synthesis of Oligonucleotides, Tetrahedron Letters 23(51), 5377 (1982)
- Matteucci et al., Synthesis of Deoxy-oligonucleotides on a Polymer Support, J. Am. Chem. Soc. 103, 3185 (1981)
- Ikehara et al., The Synthesis of Polynucleotides, Advances in Carbohydrate Chem. and Biochem. 36, 135 (1979)
- Amarnath et al., Chemical Synthesis of Oligonucleotides, Chemical Reviews 77(2), 183 ande 188-92 (1977)
- Bergstrom et al., Synthesis of C-5 Substituted Pyrimidine Nucleotides, J. Am. Chem. Soc. 98(6), 1587-89 (1976)
- Ruth et al., C-5 Substituted Pyrimidine Nucleotides, J. Org. Chem. 43(14), 2870 (1978)
- Bergstrom et al., C-5 Substituted Pyrimidine Nucleotides, J Am. Chem. Soc. 100(26), 8106 (1978)
- Bergstrom et al., Trans-5-(3,3,3-Tri-fluoro-l-propenyl)-2'-deoxyuridylate: A Mechanism-Based Inhibitor of Thymidylate Synthetase, J Medicinal Chem. 22(4), 339-40 (1979)
- Bergstrom et al., C-5-Substituted Pyrimidine Nucleotides, J. Org. Chem. 46, 1432 (1981)
- Vincent et al., Synthesis of 8-(2,4-Dinitrophenyl 2-6 aminohexyl) aminoadenosine 5'-triphosphate: Biological Properties and Potential Uses, Nucleic Acids Research 10(21), 6787 (1982)
- Langer et al., Enzymic Synthesis of Biotin-Labeled Polynucleotides:Novel Nucleic Acid Affinity Probes, Chem. Abstracts 96: 47771z (1981)
- Langer-Safer et al., Immunological Method for Mapping Genes on Drosophila Polytene Chromosomes, Chem. Abstracts 97: 88013t (1982)
- Burma, Use of Radioactive Isotopes in the Study of Nucleic Acids, Chem. Abstracts 77:72116e (1969)
- Altenburg et al., Iodine 125 in Molecular Hybridization Experiments, Chem. Abstracts 84: 27565j (1975)
- Takemura et al., Methods for Labeling of Nucleic Acids and Polynucleotides, Chem. Abstracts 91: 206523k (1977
- Sugiura et al., Methods for Enzymic Labeling of Nucleic Acids and Polynucleotides, Chem. Abstracts 91:188423w (1977)
- Bergstrom et al., Pyrido(2,3-d) pyrimidine Nucleotides, J. Org. Chem. 47, 2174 (1982)
- Nucleic Acids Res. 5(8), pp 1889-1895 (1978)
- Methods in Enzymology XXIV Part B, pp 463-475 (1974)
- Nucleic Acids Res. 11, pp 6513-6529 (1983)

## Description

The present invention relates to defined sequence single strand oligonucleotides having a length of less than 0.2 kb and which contain one or more nucleotide units to the base of which is attached a substituent group which has bound thereto one or more detectable non-radioisotopic reporter groups; such oligonucleotides being useful in the identification, localization and detection of complementary nucleic acid sequences of interest in cellular or cell-free systems.

The enzymatic production of labelled, double stranded deoxypolynucleotides has been accomplished with prior art techniques involving the incorporation of radioisotopes into double-strand DNA by the nick translation protocol of P. Rigby et al., *J. Mol. Biol.* 113: 237-251 (1977), or the gap-filling reaction described by G. Bourguignon et al,. *Virol.* 20: 290-306 (1976). Specifically, a nick is introduced via DNase, and then translated along the DNA strand using a DNA polymerase. During the nick translation procedure, DNA polymerase from E. coli (Pol I) will, in the presence of added deoxynucleoside triphosphates, condense nucleotides to the 3' hydroxyl terminus in a single strand nick region of double-stranded DNA. Simultaneously the enzyme will remove nucleotides from the 5' end of the nick. If one or more of the added triphosphates is labelled, for example, with a-³²P-phosphate, the label will be incorporated into the new strand by Pol I, following the gap-filling procedure, recessed ends left after restriction endonuclease cutting can be filled in with Klenow fragments from Pol I or T4 DNA Polymerase.

Both the nick translation and the gap-filling procedures will yield double-stranded labelled DNA. The length of the product depends upon how much DNase I is added to the reaction. This is usually optimized so that thirty percent of the label is incorporated and the strands are 400 to 800 nucleotide units in length. The product length is unpredictably heterogeneous within the 400 to 800 unit range. In order to conserve enzyme as well as labelled nucleotide, only one microgram of DNA is usually labelled in each reaction vessel.

Double-stranded polynucleotides which incorporate pyrimidine bases modified by carbon chains at the C-5 position have been prepared enzymatically in a similar manner. This has been done by enzymatic elongation of a homopolymeric primer-template as reported by J. Sagi et al., *Biochem. Biosphys. Acta*. 606: 196-201 (1980). or by nick translation/gap filling by DNA polymerases using 2-deoxyuridine 5'-triphosphate covalently linked to biotin as reported by P. Langer et al, Proc. *Nat. Acad. Sci.* USA 78:6633-6637 (1981), the biotin being capable of acting as a recognition site for avidin.

Enzymatic methods described by Rigby, et al., Bourguignon, et al, and Langer, et al, result in products having similar physical characteristics. Such enzymatically-prepared polynucleotides are 400―800 units in length, require double-stranded polynucleotides as starting materials, produce double-stranded polynucleotides in all cases, and do not allow labeling at pre-selected sites.

In addition, all enzymatic methods modify both strands of the polynucleotide. and such product strands cannot be isolated from one another. During such processes, enzymes replace either all units with modified units or, when provided with a mixture of modified and naturally-occurring nucleoside triphosphates, randomly insert modified units, Furthermore, the enzymatic process described by Langer, et al, is incapable of producing polynucleotides incorporating such reporter groups as fluorescent, luminescent, or antigenic reporter groups. None of this art is capable of producing oligonucleotides of defined length, defined sequence or single-stranded character, either with or without reporter groups. Moreover, by the prior art methods, modified bases having reporter groups attached thereto cannot be incorporated in a polynucleotide at preselected sites.

Double-stranded polynucleotides which incorporate adenine bases modified at the C-8 position have also been prepared enzymatically. This has been done by incorporating 8-aminohexylamino-ATP (a ribonucleotide) onto DNA fragments, as reported by C. Vincent et al, *Nucl. Acids Res.* 10:6787-6796 (1982). The method is limited in scope, however, allowing only 3'-end labeling with triphosphates of adenine ribonucleotides. No modified pyrimidine nucleosides can be incorporated. Furthermore, as with other enzymatic methods, both strands of a double-stranded polynucleotide are labeled and no short (<100 units) oligonucleotides of defined sequence can be produced.

The prior art enzymatic methods referred to above require the chemical synthesis of a substituted nucleoside 5'-triphosphate, and demand subsequent enzymatic recognition and incorporation of the unnatural substrate into nicked double-stranded DNA. Such methods are incapable of producing polynucleotides of any pre-selected length or sequence, and the polymerases and DNases used therein are expensive. Moreover, these methods are time consuming, inefficient, demand substantial enzymatic activity and are limited to double-stranded DNA. Only small amounts, i.e., micrograms of ill-defined polynucleotides, usually restriction fragments, are produced, and these must be tediously isolated from natural sources. Moreover, the scope of modifications obtainable in the polynucleotide product is severely limited, since the DNA polymerase cannot recognize or incorporate potentially useful reporter groups such as fluorescein or dinitrophenyl.

Attachment of one fluorosecent molecule to the 3' end of long polyribonucleotide molecules (RNA) for limited biological aplication is disclosed by J. G. J. Bauman et al, *J. Histochem. Cytochem*. 29:238 (1981). This approach also used very small amounts (microgram quantities) of RNA tediously isolated from natural sources using enzymatic methodology, and cannot be applied to DNA since both 2' and 3' hydroxyls are required therefor. The much greater chemical instability of RNA relative to DNA also minimizes the scope of application of the polyribonucleotide produced by this method.

The non-enzymatic synthesis of defined sequence oligonucleotides incorporating naturally-occurring nucleic acid bases has been reported or reviewed by S. A. Narang et al, *Meth. Enzymol* 65:610 (1980), R. Letsinger, *J. Org. Chem*. 45:2715 (1980), M. Matteucci et al. *J. Amer. Chem. Soc*. 103:3185 (1982), and G. Alvarado-Urbina et al, *Science* 214:270 (1981). Such synthesis usually involved chain extension by coupling an activated nucleotide monomer and a free hydroxyl-bearing terminal unit of a growing nucleotide chain. The coupling is effected through a phosphorus-containing group, as in the phosphate triester method reviewed by Narang et al, or one of the phosphite triester methods, Of the latter, those of Letsinger et al and Alvarado-Urbina et al use phosphochloridite chemistry, and that of Matteucci et al uses phosphoamidite chemistry.

The aforementioned chemical synthesis of oligonucleotides has incorporated only unmodified or naturally-occurring nucleic acid bases, and the end product thereof is in short fragments and resembles unmodified or naturally-occurring RNA or DNA. It is also worthy of note that the end product of such synthesis does not incorporate any labels or reporter groups.

Direct modification of homopolymeric polynucleotides has been reported in systems of polyuridylic acid [Bigge, et al. *J. Carb., Nucleosides*, *Nucleotides* 8:259 (1981)]. The reported procedure is of limited scope and is not productive of useful products. Treatments described cause extensive polynucleotide cleavage and degradation, result in product irreversibly contaminated with metal ions, and are capable of modifying only cytosine residues of a DNA polynucleotide. The method is incapable of producing defined sequence oligonucleotides of specific lengths, cannot modify thymine or purine bases, and cannot modify at previously selected sites.

From the foregoing it will be apparent that labelled, defined sequence polynucleotides have been produced heretofor only by enzymatic methods which have the disadvantages pointed out herein, particularly those relating to time, cost, product length, sequence and yield. Moreover, such methodology is productive of only double-stranded products. Such double-stranded polynucleotides can be denatured by alkali or heat to cause spontaneous short-term separation of strands in solution. However, the individual single strands cannot be physically isolated from each other, and removal of the denaturing conditions results in rapid renaturation to double-stranded form. Since conditions productive of hybridization are also productive or renaturation, subjecting the denatured polynucleotide to hybridization conditions results in return of the polynucleotide to its original double-stranded configuration in which hybridization of either of the strands to a target polynucleotide is limited by competition from the other strand.

Modification of nucleosides has been undertaken, for example, in the synthesis of antiviral C-5 substituted pyrimidine nucleosides disclosed by Bergstrom, et al, *J. Amer. Chem. Soc*. 98:1587-1589 (1976); Ruth, et al, *J. Org. Chem*. 43:2870-2876 (1978); and Bergstrom, et al, U.S. Patent Nos. 4,247,544, and 4,267,171, or in the synthesis of C-8 substituted adenine derivatives disclosed by Zappelli, et al, U.S. Patent Nos. 4,336,188 and 4,199,459. These nucleosides, and others reported by Langer, et al, and D. Ward, European Patent Application No. 0063879, are not useful in the process of the present invention. Such reported nucleosides are highly reactive at undesired sites, and, if used in oligonucleotide synthesis by chemical methods, would result in undesired side products, uncontrollable synthesis, and no desired product. Moreover, such reported nucleosides do not contain proper sites in the substituent group, cannot be modified by the attachment of reporter groups, nor do they contain masked reactive functionalities. No such nucleosides are useful in the process of the present invention.

EP 70687 states that "reagent single-stranded polynucleotides can be made by organic synthetic techniques". No examples are provided.

Adamiak, R.W. et al (Nucleic Acids Research) Vol 5, page 1889-1905, 1978, describes the chemical synthesis of an oligoribonucleotide containing N⁶-/N-threanylcarbonyl/-adenosine.

No chemical synthesis of defined sequence oligonucleotides incorporating modified bases of any kind, either with or without reporter groups, has been disclosed in the prior art.

There is an urgent need for high quality labelled, defined sequence single stand oligonucleotides which do not involve hazardous and unstable radioisotopes, and satisfaction of this need is a principal object of the present invention. This object is accomplished by a chemical, i.e., nonenzymatic, process which provides a predictable, superior product in high yield. More particularly, the process of the present invention accomplishes the chemical incorporation into defined sequence oligonucleotides of nucleotides modified with a wide variety of selected detectable non-radioisotopic reporter groups; such oligonucleotides being useful, for example, for the identification, localization, isolation and/or quantitation of complementary sequences of interest.

Another object of the invention is to provide novel nucleotides useful in the chemical synthesis of labelled, defiend sequence single strand oligonucleotides.

The process of the present invention accomplishes the novo chemical synthesis of non-radiosotopically labelled, defined sequence oligonucleotides and is superior to prior art enzymatic methods in a number of respects. More specifically the process of the present invention makes possible the synthesis of non-radioisotopically labelled, defined sequence single-stranded oligonucleotides of homogeneous predictably defined length, preferably having fewer than 200 base units, in contrast to the production of heterogeneous unpredictable populations of 400 to 10,000 base units of double-stranded character produced by prior art enzymatic methods.

The yield of product produced by the process of the present invention is of the order of hundreds to tens of thousands of micrograms, in contrast to the yield of a few micrograms provided by the prior art enzymatic methods. Moreover, the product oligonucleotides of the present invention are single-stranded, rather than the double-stranded products of enzymatic methods. The single strand configuration of the product oligonucleotides avoids the competition from a complementary strand inherent in rehybridization of double-stranded polynucleotides.

The invention comprises a homogeous defined sequence single strand oligonucleotide of less than 0.2 kb in length incorporating at least one ribonucleotide or deoxyribonucleotide unit having a pyrimidine or purine base modified at a sterically tolerant site, (such as C-5 of pyrimidines and C-8 of purines) by attachment thereto of a substituent group which has bound thereto at least one detectable non-radioistopic reporter group or solid support, the substituent group, when the modified base is a pyrimidine, incorporating a chain of two or more carbon atoms, and when the modified base is a purine, incorporating a chain of one or more carbon atoms attached to the base through a polyvalent heteroatom each such modified base being located at a preselcted position in the oligonucleotide. The invention also comprises a process for the chemical, i.e. nonenzymatic, synthesis of a defined sequence single strand oligonucleotide which comprises coupling an activated nucleotide monomer and a free hydroxyl-bearing terminal unit of a growing nucleotide chain, at least one of the monomer and terminal unit having its base modified at a sterically tolerant site by attachment thereto of a substituent group, wherein prior to or after the coupling step at least one non-radio isotopic reporter group or solid support is bound to said substituent. The invention additionally comprises novel nucleotides useful in this synthetic process.

The oligonucleotide produced by the process of the present invention may include one or more pyrimidine-based or purine-based units which may be ribonucleotides or deoxyribonucleotides, and prior to the synthesis thereof, the reporter groups are preselected, as are the particular nucleotide units to which the reporter groups are attached.

The chemical process by which the defined sequence single strand oligonucleotides of the present invention are preferably synthesized comprises coupling an activated nucleotide monomer and a free hydroxy-bearing terminal unit of growing nucieotide chain, at least one of said monomer and terminal unit having its base modified at a sterically tolerant site by attachment thereto of a substituent group capable of binding one or more detectable reporter groups.

The substituent groups of the present invention which are capable of binding reporter groups can be generally characterized as those which exhibit nucleophilic properties with respect to such reporter groups. Exemplary of such substituent groups are those which contain primary or aromatic amines, carboxylic acids, hydroxyls and the like.

The bases of the nucleotide monomer and terminal unit are selected to provide the predetermined sequence of nucleotide units desired in the end product oligonucleotide. Such bases can take the form of a the purines adenine (A), guanine (G), or hypoxanthine (H), or of the pyrimidines uracil (U), cytosine (C), or thymine (T). Such bases may also take the form of any other base which can be isolated from natural sources.

A sterically tolerant site on the nucleotide unit can be defined as position on the nucleic acid base of the unit at which modification of the unit can be effected by attachment thereto of the substituent group without causing significant interference with hybridization of the product oligonucleotide and complementary nucleic acid components, and without sterically preventing the substituent group from binding one or more reporter groups. Sterically tolerant sites are found at the C-8 position of purines and at the C-5 position of pyrimidines. Since the oligonucleotides of the present invention are particularly useful as hybridization probes, modifications which attach substituent and/or reporter groups should not be at sites on the pyrimidine or purine bases which are necessary for specific hybridization. Sites which sould not be modified include N¹ and N⁶ of adenine bases; N¹, N², and O⁶ og guanine bases) N³ and N⁴ of cytosine bases. Generally, substitution at any heteroatom (N or O) should be avoided.

A reporter groups can be defined as a non-radioistopic chemical group which may be aromatic and/or polycyclic and which has a physical or chemical characteristic which can be readily measured or detected by appropriate physical or chemical detector systems or procedures. Reporter groups which are useful in oligonucleotides of the present invention are readily detectable. Ready detectability may be provided by such characteristics as color change, luminescence, fluorescence; or it may be provided by the ability of the reporter group to serve as a ligand recognition site. Such groups are termed functionally colorimetric, luminescent, fluorescent or ligand recognition groups. Among such groups are those suitable for ready detection by conventional detection techniques, such as colorimetric, spectrophotometric. fluorometric detection, as well as those which are capable of participating in the formation of specific ligand-ligand complexes which contain groups detectable by such conventional detection procedures.

A reporter group as used herein may be characterized as a non-radioistopic label which has physical, chemical or other characteristics which can be readily measured or detected by the use of appropriate measurement or detection procedure. A reporter group as defined herein also includes ligand recognition groups which are capable of initiating one or more ligand-ligand interactions which ultimately provide a reaction product or a complex having physical, chemical or other characeristics which can be readily measured or detected by the use of appropriate measurement or detection procedures.

Exemplary of the measurable or detectable characteristics which such groups, reaction products or complexes may exhibit or induce are a color change, luminescence or fluorescence. Such characteristics can be measured or detected by the use of conventional colorimetric, spectrophotometric or fluorometric sensing instrumentation.

The interactions which usefully can be initiated by the reporter group defined herein include appropriately specific and selective ligand-ligand interactions productive of groups or complexes which are readily detectable, for example, by colorimetric, spectrophotometric or fluorometric, detection procedures. Such interactions may take the form of protein ligand, enzyme-substrate, antibody-antigen, carbohydrate-lectin, protein-cofactor, protein-effector, nucleic acid-nucleic acid or nucleic acid-ligand interactions. Exemplary of such ligand-ligand interactions are dinitrophenyl-dinitrophenyl antibody, biotin-avidin, oligonucleotide-complementary oligonucleotide, DNA-DNA, RNA-DNA and NADH-dehydrogenase. Either one of each such ligand-ligand pairs may serve as a ligand recognition type reporter group. Other useful interactions will suggest themselves to those skilled in the art.

In the process of the present invention, a selected non-radioisotopic reporter group or groups can optionally be attached to the nucleotide monomer before coupling of the monomer to the terminal unit of the nucleotide chain, or it can be attached to the product oligonucleotide after formation thereof. The sequence of the nucleotide units in the product oligonucleotide is preselected to provide such oligonucleotide with precise specificity for its ultimate use. The oligonucleotides of the present invention are useful tools in recombinant DNA and other protocols involving nucleoic acid rehybridization techniques. Among such uses are identification, localization, isolation and/or quantitation of complementary sequences of interest in cellular or cell-free systems. More specifically, such uses may include diagnostic applications or any fundamental biological event involving hybridization of nucleic acid components, or purification of complementary sequences by affinity chromatography when the product oligonucleotide is attached to a solid support through the modifications at a sterically tolerant site, with or without subsequent detection.

The nucleotide units in the product oligonucleotide may be purine or pyrimidine-based and may comprise units having naturally-occurring bases intermixed with units having modified bases. Such units may be ribonucleotides or deoxyribonucleotides. The coupling step preferably involves coupling of a monomer unit activated at the 3' position with a free 5' hydroxyl of the terminal unit of the growing nucleotide chain. Alternatively such coupling can involve coupling of a monomer unit activated at the 5' position with a free 3' hydroxyl of the terminal unit of the nucleotide chain. The terminal unit may be the initial or only unit in the growing nucleotide chain at the time of coupling thereto of the nucleotide monomer, or it may be the terminal one of a plurality of nucleotide units.

The process of the present invention produces defined sequence oligonucleotides of the following generic formula:

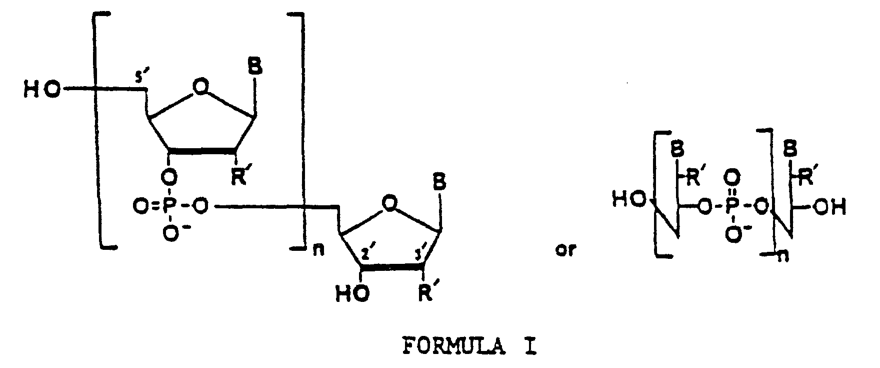

Wherein n is 1 to about 199, preferably about 5 to about 60, and most preferably about 10 to about 40, R' is hydrogen or hydroxy, and B is any of the naturally-ccurring purine or pyrimidine bases adenine, guanine, cytosine, uracil, thymine, or any other naturally-occuring base, the nucleotide units having naturally-occurring bases being independently intermixed with one or more nucleotide units having modified bases (B^{m}). The modified pyrimidine bases (Py^{m}) are substituted at the C-5 position, and typical examples thereof are the uracil and cytosine bases illustrated by the following generic formulas:

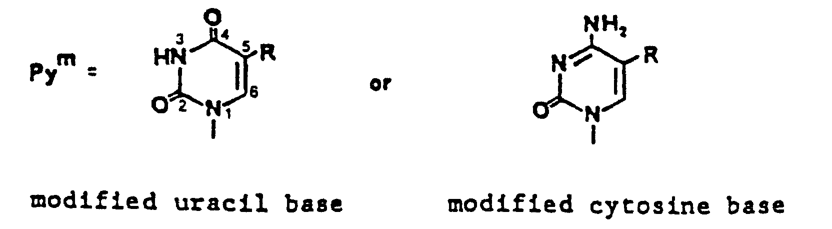

The modified purine bases (Pu^{m}) are substituted at the C-8 position. and typical examples thereof are the modified adenine and guanine bases illustrated by the following generic formulas:

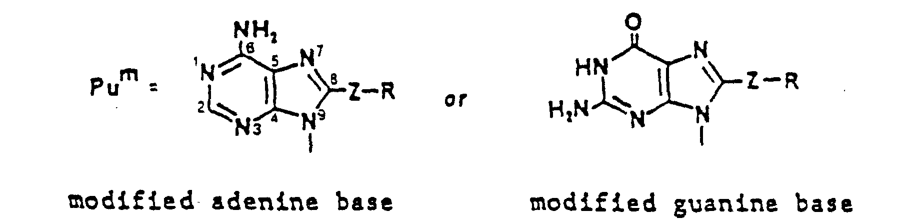

The substituent group R is characterized by its ability to bind one or more reporter groups. In the modified pyrimidine bases the substituent group R comprises two or more caroon atoms, whereas in the modified purine bases R comprises one or more carbon atoms. In this context, R preferably takes the form of one of the following functionalized carbon chains:

R = -CH=CR₁R₂, -CH₂CHR₁R₂, -CHR₁R₂,

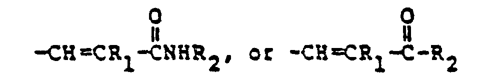

wherein R₁ is hydrogen or alkyl; R₂ is alkyl, alkenyl, aryl, or functionalized alkyl, alkenyl, aryl wherein functional groups include one or more amines, amides, nitriles, carboxylic acids and esters, hydroxy, dinitrophenyl, aminobenzenesulfonates, or the like; and Z is a polyvalent heteroatom such as nitrogen, oxygen or sulfur. In addition, R₂ may be attached to a solid support, or to one or more reporter groups which function, for example, as a colorimetric, fluorescent, luminescent, or ligand recognition group. Functionally fluorescent groups include fluoresceins, rhodamines, and the like or adducts thereof including fluorescamine; functionally luminescent groups include luminols, acridines, luciferins, dioxetanes, dioxamides, and the like or adducts thereof. Ligand recognition groups include vitamins (such as biotin or adducts thereof including iminobiotin and desthiobiotin), antigens such as dinitrophenols, carbohydrates and other functional groups or adducts of such groups which can be recognized by ligand-like interactions with proteins, or from which such ligand-like interactions can be elicited. Another oligonucleotide capable of interaction with nucleic acids is illustrative of a group from which a ligand-like interaction can be elicited. Ligand recognition groups may also serve as functionally colorimetric reporter groups when recognition results in dye formation. For example, when dinitrophenyl is used as a reporter group, known detection systems using an antidinitrophenyl antibody coupling to peroxidase can be used as a detection system, resulting in a color change.

When reference is made herein to the use of purine or pyrimidine bases. such expressions are intended to include analogs of such bases. Among such an analogs are the analogs of purine bases, such as the deazaadenosines (tubercidins, formycins, and the like), and the analogs of pyrimidine bases, such as deazauracil, deazacytosine, azauracils, azacytosines, and the like.

Oligonucleotides of Formula I are best prepared by chemical synthesis from monomer nucleotide analog units of the formula: 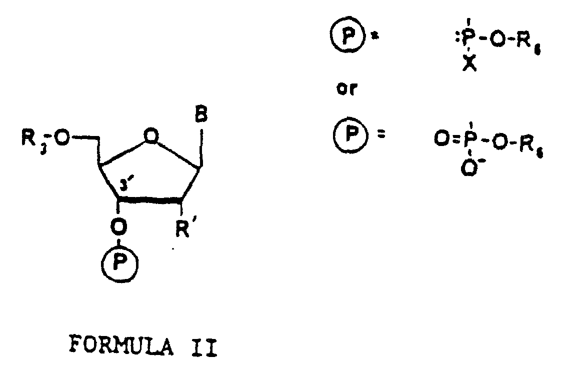 where:
- R₆ =: methyl, chlorophenyl
- X =: chloro, dialkylamino, morpholino
wherein R₃ is trityl (triphenylmethyl), dimethoxytrityl or other appropriate masking group for the 5'-hydroxyl; B and R' are masked, if appropriate: and Ⓟ represents a phosphorus-containing grouo suitable for internucleotide bond formation during chain extension in synthesis of a product oligonucleotide. The phosphorus-containing groups Ⓟ suitable for internucleotide bond formation are preferably alkyl phosphomonochloridites or alkyl phosphomonoamidites. Alternatively phoshate triesters may be employed for this purpose. The monomer unit may alternatively have R₃ attached at the 3' hydroxyl and Ⓟ attached at the 5'-hydroxyl.

Generally, the term "masking group" or "blocking group" is a functional expression referring to the chemical modification or "blocking" of an integral functional group by attachment of a second moiety to disguise the chemical reactivity of the functional group and prevent it from reacting in an undesired manner. Such modification is reversible, and allows subsequent conversion back to the original functional group by suitable treatment. In many cases, such masking formally interconverts structural functionality, e.g., a primary amine masked by acetylation becomes a substituted amide which can be later converted back to the primary amine by appropriate hydrolysis.

The compounds of Formula I include the acceptable conjugate acid salts thereof. Conjugate acids which may be used to prepare such salts are those containing nonreactive, cations and include, for example, nitrogen-containing bases such as ammonium salts, mono-, di-, tri- or tetra-substituted amine salts, and the like, or suitable metal salts such as those of sodium, potassium, and the like.

The process steps of the present invention will now be generally described and illustrated diagrammatically. Thereafter, the invention will be illustrated more specifically and detailed examples thereof provided. Since the invention relates to oligonucleotides incorporating both pyrimidine-based and purine-based nucleotide units, the use of both pyrimidine and purine-based compounds in the synthetic process will be illustrated. The specific pyrimidine and purine-based compounds illustrated are only exemplary of the respective pyrimidine and purine classes, and it is to be understood that any other member of the respective class can be substituted therefore in the process and the product oligonucleotide. whenever suitable or desired. While deoxyribonucleotide compounds are shown for the most part, it is understood that ribonucleotide compounds wherever ribonucleotide compounds are desired in the product oligonucleotide.

The following class of nucleosides is essential as intermediates in the process for synthesizing the new oligonucleotides of the invention. Such nucleotides each have a base which is modified by a substituent group comprising a functionalized carbon chain and one or more amides, the nitrogen of the amides being attached to a sterically tolerant site on the base through the carbon chain. In the case of pyrimidine-based nucleosides, the carbon chain is attached at the C-5 position, and in the case of the purine-based nucleosides, the carbon chain is attached at the C-8 position through a polyvalent heteroatom, such as nitrogen, oxygen or sulfur. In addition, such nucleosides are chemically blocked at the 5' position (or the 3' position) with a group, such as dimethoxytrityl, appropriate for the chemical synthesis of oligonucleotides.

In this class of nucleosides the substituent group may be chosen from -CH₂CHR₁CₙH₂ₙY, -CH=CR₁CₙH₂ₙ Y, 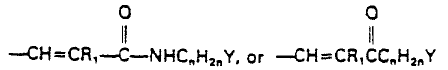 wherein R₁ is hydrogen or C₁₋₆ lower alkyl, n is 0 to 20 and Y is one or more amido, substituted amido, substituted amino or substituted aminoalkylphenyl groups. More specifically, Y may include one or more 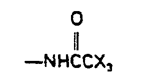 wherein X is hydrogen, fluorone or chlorine. Synthesis of these nucleosides, as well as of the masked forms thereof, is described hereinafter in Examples I, II, IV, VI, VII, VIII, X, XII, and XIII. Preferred nucleosides incorporate with substituent group 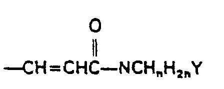 at the C-5 of pyrimidine nucleosides wherein n = 3 to 12 and Y is 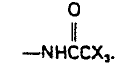 Most preferred are such nucleosides wherein the pyrimidine base is uracil.

The process of the present invention may be initiated by the preparation of the selected nucleoside. Generally, the most preferred nucleotides are best prepared in the following manner. 5-(Methyl 3-acrylyl)-2'-deoxyuridine is prepared from 2'-deoxyuridine by the method of Bergstrom and Ruth [*J. Amer. Chem. Soc*. 96: 1587 (1976)]. The nucleoside is then treated with 1.05 equivalents of dimethoxytrityl chloride in pyridine for 4 hours to block the 5' hydroxyl with dimethoxytrityl (DMT). The resulting product is purified by silica chromatography eluting a gradient of 0-10% methanol in chloroform containing 2% triethylamine. The purified 5'-DMT-5-(methyl 3-acrylyl)-2'-deoxyuridine is treated with 1 N KOH for 24 hrs. at ambient temperature to hydrolyze the methyl ester.

The resulting 5'-DMT-5-(3-acrylyl)-2'-deoxyuridine is treated with excess dicyclohexylcarbodiimide and hydroxybenztriazole in pyridine. After 4 hours, a 2-5 fold excess of 1,5-diaminoheptane is added, and the reaction stirred overnight. After 12-20 hours, a 10-20 fold excess of trifluoroacetic anhydride is added, and the reaction stirred at room temperature for 4 hours. The product is purified by silica chromatography eluting a gradient of 0-10% methanol in chloroform containing 2% triethylamine, followed by exclusion chromatography using Sephadex LH-20 eluting 1% triethylamine in methanol. Appropriate fractions are combined to yield 5'DMT-5-[N-(7-trifluoroacetylaminoheptyl)-1-acrylamido]-2'-deoxyureidine; such product is appropriate for oligonucleotide synthesis by the phosphochlorodite procedure described in Examples XV and XVIII. Alternatively, such a compound can be prepared by the combination of methods described in Examples II and III. Replacing diaminophetane in this process with other diaminoalkanes (e.g., diaminopropane, diaminohexane, diaminododecane) is productive of the other compounds of varying substituent length wherein n = 3, 6, or 12 and 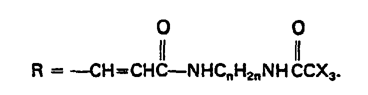 Two such nucleosides, one pyrimidine (uracil)-based and the other purine (adenine)-based, are shown at the top of the diagram below illustrating the process. Reactive sites on the base of the nucleosides are then masked, as shown in Reaction 1, by attachment of, for example, a benzoyl group (Bz) to the amine at the 6 position of the adenine-based nucleoside. Such masking is generally described in "Synthetic Procedures in Nucleic Acid Chemistry", Vol. 1, W. Zorbach and R. Tipson eds. (Wiley - Interscience, N.Y., 1968). Unprotected amines on the substituent group are masked, for example, by attachment thereto of trifluoroacetyl groups (Ac), as also shown in Reaction 1.

The selected 3' or 5' hydroxyl of the nucleoside is then masked by attachment thereto of a dimethoxytrityl (DMT) group. In Reaction 2 illustrated below, the 5'-hydroxyl is masked, leaving the 3' hydroxyl free or available for reaction. Alternatively, the 3'-hydroxyl could be masked, leaving the 5' hydroxyl free.

The nucleoside is then converted to an activated nucleotide monomer, preferably by attahcement to its 3' hydroxyl of a phosphorus-containing group which includes an activating moiety. When the modified nucleoside is properly blocked, modifications of the procedures described by Letsinger, et al, Matteucci, et al, or as reviewing by Narang, et al can be utilized for oligonucleotide synthesis. The use of phosphochloridite chemistry such as that disclosed by Letsinger, et al., is detailed in Examples XVI-XVIII. In order to use phosphamidite chemistry, a modification of the procedure of Matteucci, et al, is used, phosphitylating the protected modified nucleoside with methyl chloro (N, N-diisopropyl)phosphoamidite or methyl chlorophosphomorpholidite, as in the improved procedure of Dorper, et al [*Nucleic Acids Res*. 11:2575(1983)]). Alternatively, the protected modified nucleoside can be phosphorylated with 1.2 eq. chlorophenyl dichlorophosphate in trimethylphosphate at room temperature followed by quenching with water to give the 3'-chlorophenyl phosphate adduct of the modified nucleoside, such adducts being useful in a modification of the phosphotriester approach as illustratively reviewing by Narang, et al. The diagram illustrates in Reaction 3 the synthesis of activated monomer nucleotide units of Formula II by attachment to the nucleoside 3' hydroxyl of a phosphomonochloridite group in which the chlorine functions as an activating moiety.

Coupling or condensation of the selected activated nucleotide monomer, i.e. the uracil-based monomer or the adenine-based monomer, to the terminal unit of a growing nucleotide chain is illustrated in Reaction 4 in the diagram. The nucleotide chain is shown as including in its right hand end a nucleotide unit having a naturally occurring base and having a solid support or masking group R₄ attached to its 3' hydroxyl. The illustrated chain also includes one or more (n') nucleotide units having naturally-occurring bases, said units being couled to the 5' hydroxyl of the nucleotide unit, the terminal one of the nucleotide units having a free hydroxyl at the 5' position. In the coupling reaction the chlorine of the monomer reacts with the hydrogen of the free hydroxyl of the terminal unit and is displaced, so that the oxygen of the terminal unit couples to the phosphorus of the monomer as shown, and the monomer thereby becomes the new terminal unit of the nucleotide chain.

The DMT 5' blocking group is then removed to permit further extension of the nucleotide chain by sequential coupling thereto of additional activated nucleotide monomer units. The nucleotide units added to the chain can be preselected and may have either naturally-occurring or modified bases. The diagram shows in Reaction 4a the further extension of the chain by the addition of one or more (n") nucleotide units having naturally-occurring bases.

When an oligonucleotide of the selected length and sequence has been synthesized, the DMT group is removed from the terminal unit thereof, and the masked reactive groups are unmasked. Examples of modified uracil and adenine bases with their reactive groups unmasked are also shown diagrammatically at Reaction 5. If the initial nucleotide unit of the chain is bound to a solid support R₄, the chain is then removed from such solid support. The appropriate order of unmasking can be preselected.

Reporter groups R₅ appropriate for the intended use of the product oligonucleotide can then be bound to such substituent groups as exemplified in Reaction 6, which illustrates the respective bases with reporter groups R₅ bound to the respective substituent groups thereof. 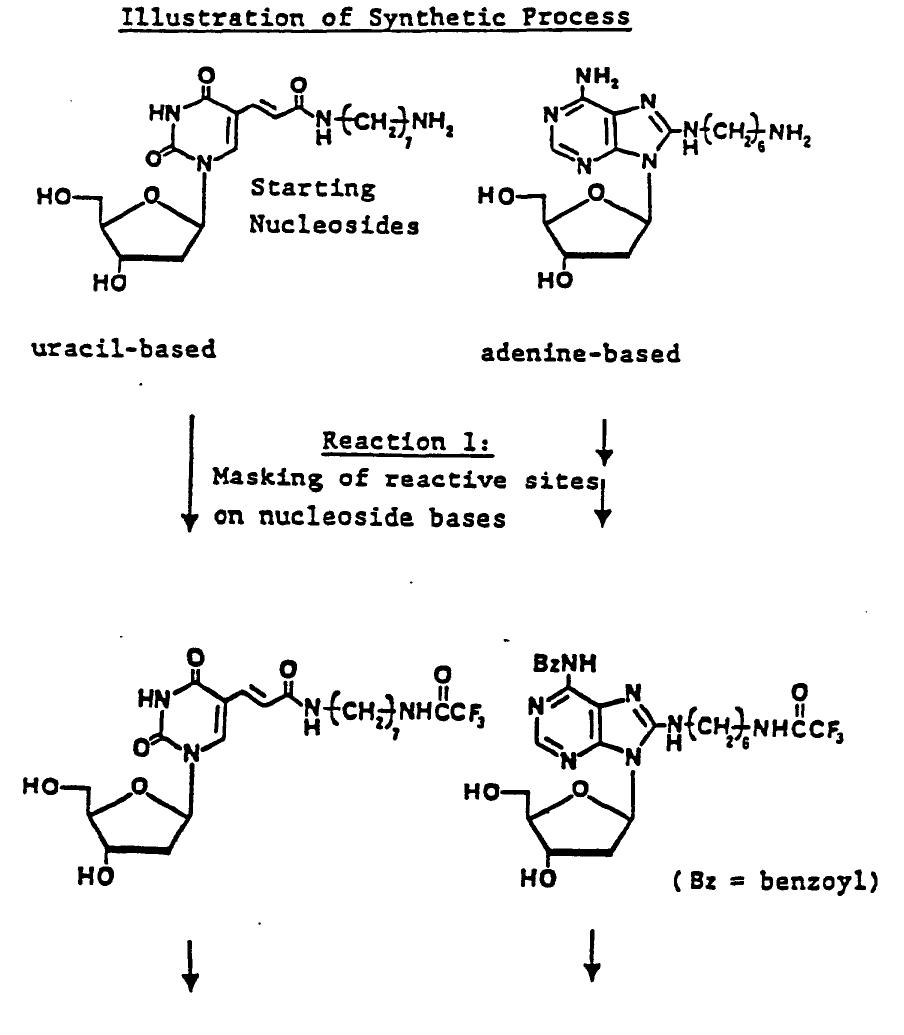 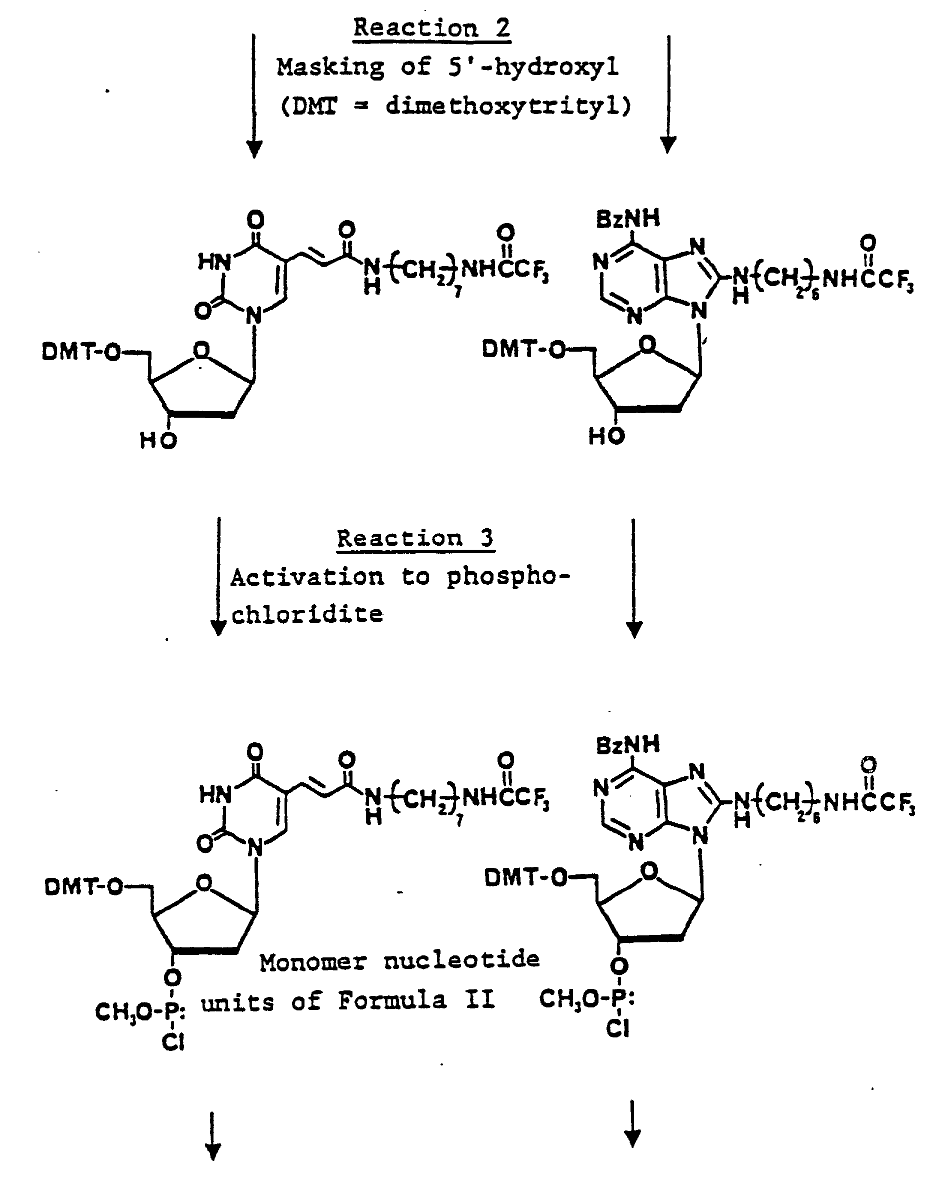 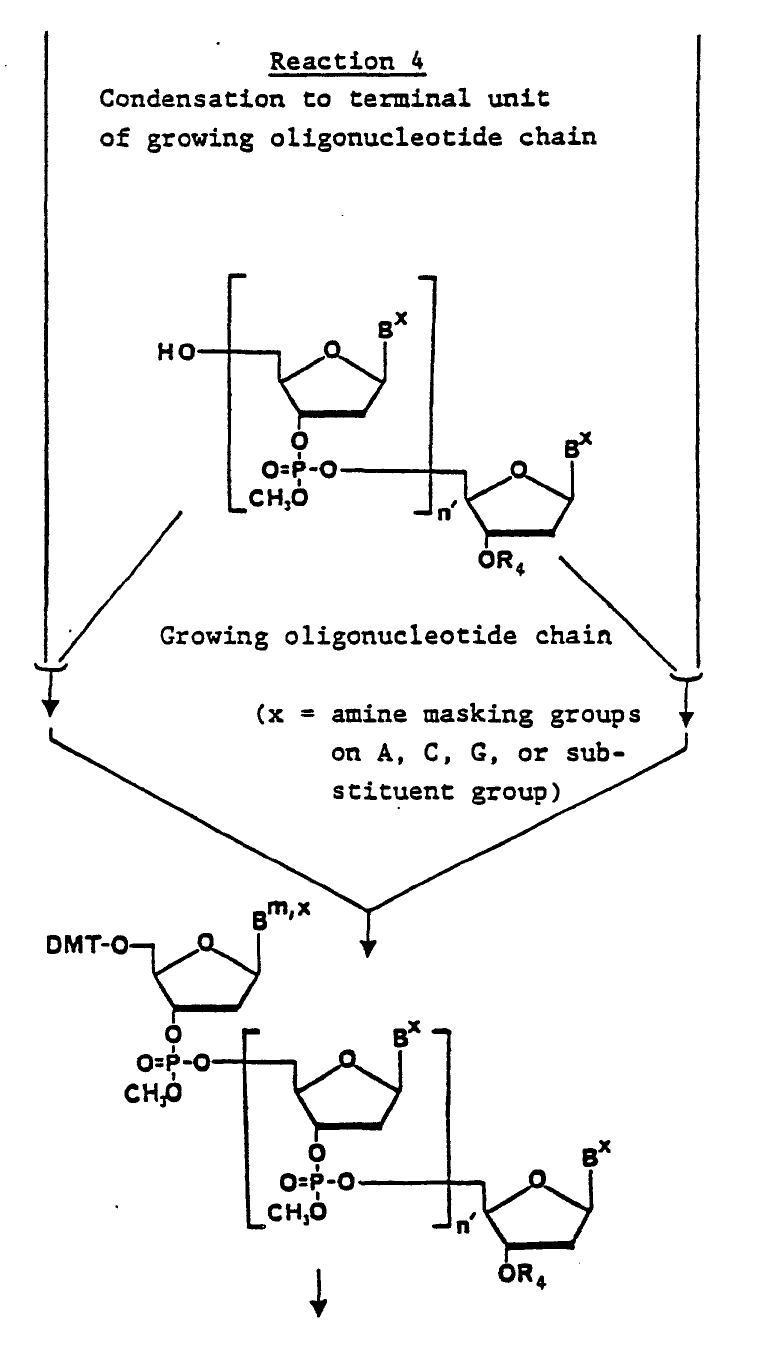 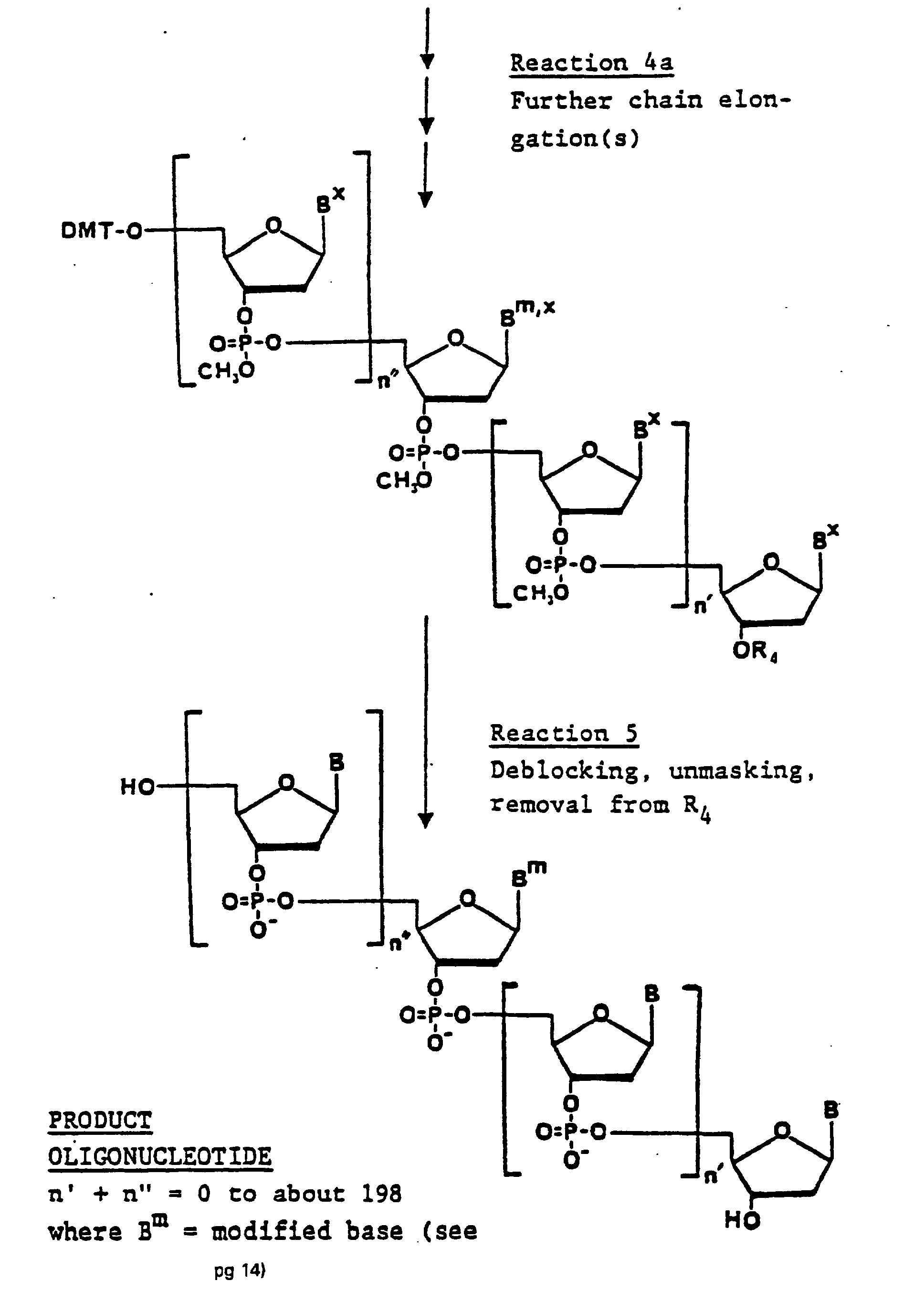 where, for example, B^{m} in product oligonucleotide (pg 13) is: 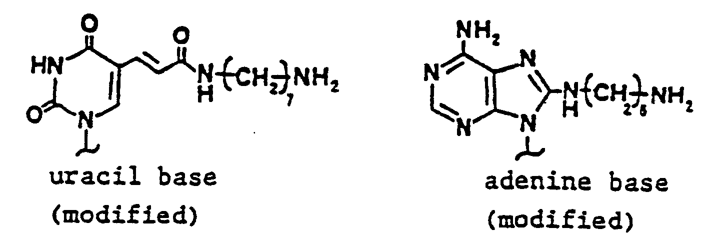 To the product oligonucleotide a variety of useful reporter groups (R₅) may be attached. For example:

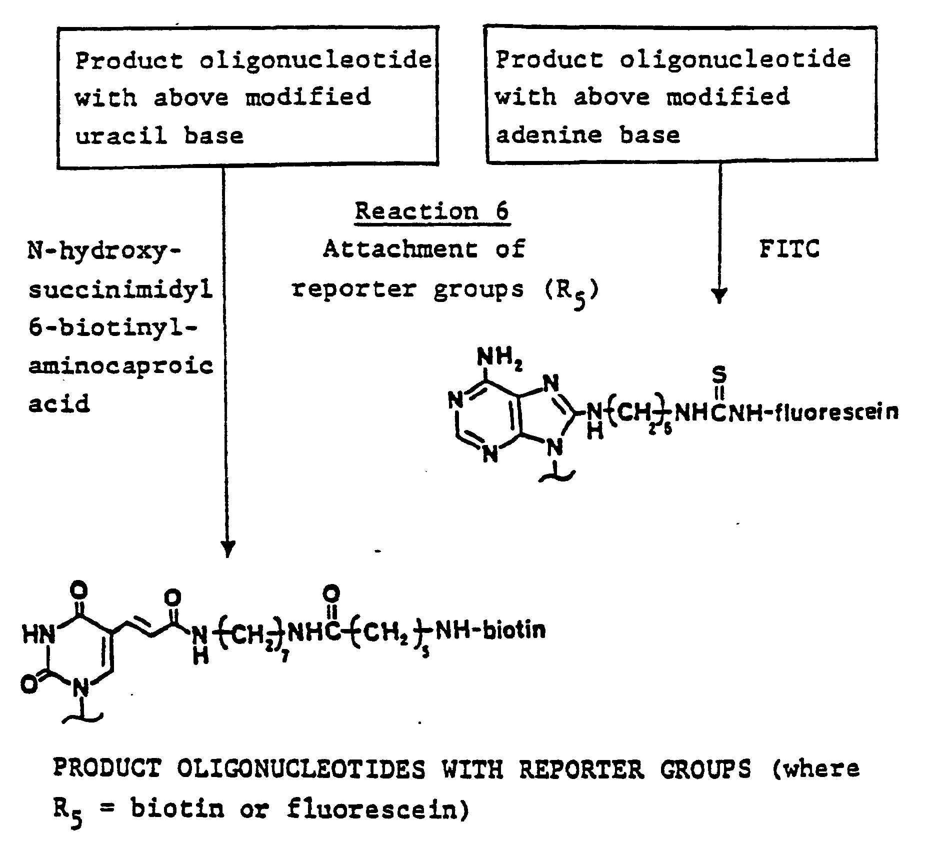

Having discussed the process of the present invention in general terms and illustrated the same diagrammatically, each of the reactions referred to will now be discussed more specifically. With reference to Reaction 1, masking of chemically reactive amines such as N⁴ of cytosine, N⁶ of adenine, N² of guanine, and alkyl or aryl amines of the modified baes with suitable masking groups can be conveniently accomplished in suitable solvents such as alcohols, pyridines, lutidines, chloroform, and the like, by reaction of the nucleosides with an excess of appropriate acid anhydrids for about 1 to 24 hours at temperatures in the range of 0°C to 110°C, generally 20°C to 80°C. Appropriate acid anhydrides include acetic anhydride, trifluoroacetic anhydride, benzoyl anhydride, anisoyl anhydride, and the like. Preferred are acetyl, trifluoroacetyl, benzoyl, and isobutyryl anhydride.

Masking of the 5'-hydroxy in Reaction 2 can be conveniently effected by reaction of the nucleosides with a slight excess of appropriate acid-labile masking reagents, such as tritylchlorides, monomethoxytrityl chloride, dimethoxytrityl chloride (DMTCI), trimethoxytrityl chloride and the like. Preferred is dimethoxytrityl chloride. Typical reactions are carried out in suitable solvents, such as pyridine, lutidines, trialkylamines, and the like, at temperatures in the range of -20°C to 120°C. generally 20°C to 100°C, for about 1 to 48 hours. The preferred reaction utilizes 1.1 equivalents of DMTCI in pyridine at room temperature for 2 hours.

It is generally preferred that the respective products of each reaction described hereinabove by separated and/or isolated prior to use as a starting material for a subsequent reaction. Separation and isolation can be effected by any suitable purification procedure such as, for example, evaporation, filtration, crystallization, column chromatography, thin layer chromatography, etc. Specific illustrations of typical separation and isolation procedures can be had by reference to the appropriate examples described hereinbelow; however, other equivalent separation procedures can, of course, also be used. Also, it should be appreciated that, where typical reaction conditions (e.g., temperatures, mole ratios, reaction times) have been given, conditions both above and below the typical ranges can also be used, though generally less conveniently.

Activation to the phosphite analog illustrated in Reaction 3 can be most conveniently effected by treatment of the nucleoside compounds with suitable phosphitylating agents in appropriate solvents at temperatures in the range of -90°C to 60°C for 1 minute to 2 hours. Suitable phosphitylating agents include methylphosphodichloridite, o-chlorophenylphosphodichloridite, p-chlorophenylphosphodichloridite, methylphospho(dialkylamino) monochloridite, and the like. Appropriate solvents include pyridine, lutidines, acetonitrile, tetrahydrofuran, dioxane, chloroform and the like containing 0-20% appropriate base (generally 1-5 vol %) such as lutudines, collidines, trialkylamines and the like. Preferred phosphitylating agents are methylphosphodichloridite, o-chlorophenylphosphodichloridite, and methylphospho(di-isopropylamino)-monochloridite. Preferred phosphytilating conditions are with 0.9 equivalents of methyl phosphodichloridite in pyridine or acetonitrile containing 5% 2,6-lutidine for 5 to 10 minutes at room temperature or below.

The chemical incorporation of the modified nucleotide analog monomers into a growing nucleotide chain to produce defined sequence nucleotides is illustrated in Reactions 4 and 4a. Typical condensations are in appropriate solvents at temperatures in the range of -20°C to 50°C, preferably at ambient temperature, for about 1 to 60 minutes. Appropriate solvent mixtures include pyridine, lutidines, acetonitrile, tetrahydrofuran, dioxane, chloroform and the like containing 0-20% appropriate base (generally 1 to 5 volume %) such as lutidines, collidines, trialkylamines and the like. The growing chain may be soluble, insoluble, or attached to a suitable solid support by appropriate chemical methods known in the art. Preferred is attachment to a solid support. Furthermore, the growing chain may or may not have previously incorporated one or more modified nucleoside analogs.

After condensation of the activated monomer to the growing chain, in Reaction 4, the initial product is treated with suitable reagents to accomplish oxidation of the intermediate phosphitetriester, optional capping to block unreacted 5'-hydroxyls on the oligonucleotide chain, and removal of the 5'-DMT group. Oxidation of the phosphite triester can be accomplished by treatment with 0.1-5 w/vol % iodine in suitable solvents, for example, tetrahydrofuran/water/lutidine mixtures. Chemical capping of unreacted 5'-hydroxyls can be accomplished by acetylation or acylation with, for example, acetic anhydride and 4-dimethylaminopyridine in tetrahydrofuran/lutidine mixtures. Removal of the 5'-blocking group, usually DMT, is most conveniently effected by treatment with mild organic acids in nonprotic solvents, such as mild acids including, for example, 1-5 vol % dichloroacetic or trichloroacetic acid in chloroform or dichloromethane. The growing nucleotide chain, after removal of DMT, can now serve as acceptor for subsequent elongation by sequential reaction with activated monomers to eventually produce the oligonulceotide of desired length and sequence, as shown in Reaction 4a.

After an oligonucleotide of desired sequence is produced, Reaction 5 is accomplished to provide the product oligonucleotide. To this end, thiophenol treatment is used to remove methyl masking groups from phosphate triesters, and suitable aqueous alkali or ammonia treatment is used to remove benzoyl, acetyl, isobutyl, trifluoroacetyl, or other groups from the protected amines and/or to remove the product from the solid support. Removal of DMT from oligonucleotide product is accomplished by the appropriate treatment with a mild acid, such as aqueous acetic acid at ambient temperature to 40°C for 10 to 60 minutes. Such reactions may be accomplished before or during final purifications. Final purification is accomplished by appropriate methods, such as polyacrylamide gel electrophoresis, high pressure liquid chromatography (HPLC), reverse phase or anion exchange on DEAE-cellulose, or combinations of these methods.

The process described herein for synthesis of oligonucleotides can utilize modified deoxyribonucleosides (where R' is H) or modified ribonucleosides (where R' is hydroxyl). When ribonucleosides are used, the 2'-hydroxyl is masked by an appropriate masking group such as, for example, that afforded by silylethers. Other ribose analogs, including arabinose and 3'-deoxyribose, can also be accommodated in the process to produce the desired oligonucleotide.

The substituent group modifying a nucleotide base must be capable of binding one or more reporter groups either prior to or after the chain extension coupling reaction. In the latter case, selected product oligonucleotides are reacted with suitable agents to attach such reporter groups. For example, when modified bases are incorporated into the oligonucleotide and R₂ of the substituent group contains one or more primary amines, coupling with amine-reactive groups such as isocyanate, isothiocyanate, active carboxylic acid conjugates, epoxides or active aromatic compounds using suitable conditions is productive of amide, urea, thiourea, amine or aromatic amine linkages. For example, an oligonucleotide which contains a uracil or adenine base modified by a substituent group having a primary amine, as shown in the Reaction 5 diagram, can be reacted with a suitable reagent, such as fluorescein isothiocyanate or N-hydroxysuccinimidyl 6-biotinylaminocaproic acid, to provide a reporter group R₅ (fluorescein or biotin, respectively) bound to the substituent group as shown in Reaction 6. Other reporter groups which can be attached in similar manner include a wide variety of organic moieties such as fluoresceins, rhodamines, acridinium, salts, dinitrophenyls, benzenesutfonyls, luminols, luciferins, biotins, vitamins, carboxyhydrates and the like. Suitably active reporter groups are available commercially, or can be synthesized, for example, by processes of the type generally described in "Bioluminescence and Chemiluminescence" [M. DeLuca and W. McElroy, eds., Acad, Press, New York (1981)], by D. Roswell, et al., or H. Schroeder, et al. [*Meth. Enzymol,* LXUII, 1978], and references cited therein.

Typically, attachment of reporter groups is conveniently accomplished in predominately aqueous solvents by reaction of the substituent groups of modified bases wherein R₂ = Cₙ H₂ₙ NH₂ with excess of the selected reporter group at temperatures in the range of about -20°C to 50°C (preferably 20°C to 40°C) for 1 to 24 hours. Suitable solvents are an aqueous buffer and 0-50% organic solvents such as lower alcohols, tetrahydrofuran, dimethylformamide, pyridine, and the like. Preferred reporter group reactants include fluorescein isothiocyanates, dinitrophenylisothiocyanates, fluorodinitrobenzene, N-hydroxysuccinimidylbiotin, N-hydroxysuccinimidyl dinitrobenzoate, isothiocyanates such as aminobutyl ethyl isoluminol isothiocyanate and the like, active esters of carboxyfluoroscein, fluoroescamine, rhodamine, biotin adducts. dioxetanes, dioxamides, carboxyacridines, carbohydrates and the like.

Additionally, when the product oligonucleotide includes modified bases wherein R contains one or more carboxylic acids, mild condensations with, for example, primary alkylamines is productive of amide linkages. Typically, this is conveniently effected in predominately aqueous solvents by reaction of the oligonucleotide with excess reporter group which contains a primary amine in the presence of suitable condensing agents, such as water-soluble carbodiimides, at temperatures in the range of about -20°C to 50°C (preferably 20°C to 40°C) for 6 to 72 hours. Preferred reporter groups of this class include (aminoalkyl)-amino-naphthalene-1, 2-dicarboxylic acid hydrazides, amino-fluoresceins, aminorhodamines, aminoalkyl luminols, aminoalkylaminobenzenesulfonyl adducts, amino sugars, and the like. Furthermore, the chemical synthesis of the initial oligonucleotide product may be accomplished with modified nucleotide monomers wherein the substituent group has attached thereto such reporter groups. If such reporter groups require masking, they are appropriately masked during the coupling reaction. On the other hand, certain reporter groups do not require masking. For example, nitrophenyl adducts without masking may be present on the modified nucleotide monomer during the coupling reaction without adverse affect.

Reporter groups useful in the method of this invention generally include aromatic, polyaromatic cyclic, and polycyclic organic moieties which are further functionalized by inclusion of heteroatoms such as nitrogen, oxygen, sulfur and the like.

Product oligonucleotides can include more than one type of modification or more than one modified base. An illustrative example of an oligonucleotide of this type is one of the structure: 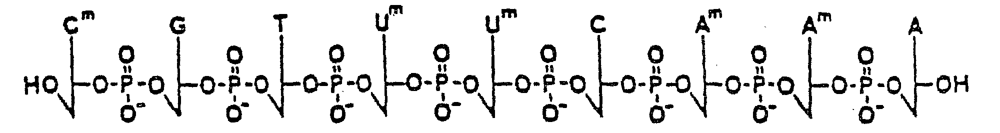 wherein C^{m} is 5-(3-aminopropyl) cytosine, U^{m} is 5-[N-(4-aminobutyl)-1-acrylamido]uracil and A^{m} is 8-[6-2,4-dinotrophenyl)-aminohexyl]aminoadenine. This product is further modified by reaction with fluorescein isothiocyanate to provide a fluorescein reporter group on C^{m} and U^{m}.

Such a product oligonucleotide illustrates the variety of the selection of modified and unmodified nucleotide units in a product oligonucleotide made possible by the process of the present invention. More specifically, such oligonucleotide illustrates the use of more than one type of nucleotide unit having its base modified by subsequent groups having attached thereto reporter groups providing the same or different types of reporter group function. Also illustrated are units whose bases are modified by subsequent groups to which reporter groups are bound, i.e., C^{m} and U^{m}, whereas A^{m} is illustrative of a unit whose base is modified by a substituent group to which is attached a dinitrophenyl group. Such oligonucleotide additionally illustrates that it can include more than one nucleotide unit of the same type, and that it can include units having unmodified bases intermixed with units having modified bases.

Instead of attaching reporter groups to the primary amines of the substituent groups as illustrated in Reaction 6, such amine or other group can alternatively be coupled to suitably activated solid supports. This produces a defined sequence oligonucleotide which is convalently bound to such supports through the modified bases. Such solid supports are useful in the detection and isolation of complementary nucleic acid components. Alternatively, the modified nucleoside monomers can be coupled to solid supports prior to the chain extension coupling Reaction 4, to thereby provide solid supports for such monomers during the coupling reaction.

The following specific examples are provided to enable those skilled in the art to practice the invention. The examples should not be considered limitations upon the scope of the invention, but merely as being illustrative and representative thereof. To aid in structural clarification, references are made to the reactions illustrated in the aforementioned process diagram.

### Example I

This example illustrates the synthesis of a modified nucleoside precursor 5-(3-trifluoroacetylaminopropenyl)-2'-deoxyuridine.

5-Chloromercuri-2'-deoxyuridine (3.6 g, 7.8 mmol) is suspended in 200 ml methanol. N-Allyltrifluoroacetamide (6.8 ml, 55 mmol) is added, followed by addition of 41 ml of 0.2 N lithium tetrachloropalladate in methanol. After 18 hours stirring at room temperature, the reaction is gravity filtered to remove the black solid palladium, and the yellow methanolic filtrate is treated with five 200 mg portions of sodium borohydride, then concentrated under reduced pressure to solid residue. The residue is purified by flash column chromatography on silica gel eluting 15 vol% methanol in chloroform. Appropriately pure fractions of product are combined and concentrated under reduced pressure to give crystalline 5-(3-tritluoroacetylaminopropenyl)-2'-deoxyuridine (2.4 g). UV λₘₐₓ 291 nm (ε 7800), λₘᵢₙ 266 nm, (ε 4400); TLC (silica eluting 15 vol% methanol in chloroform) R_{f} = 0.4.

### Example II

This example illustrates the synthesis of a modified nucleoside precursor 5-[N-(trifluoroacetylaminoheptyl)-1-acrylamidol-2'-deoxyuridine.

5-Chloromercuri-2'-deoxyuridine (3.6 g, 7.8 mmol) is suspended in 200 ml methanol. N-(7-trifluoroacetylaminoheptyll-acrylamide (55 mmol) is added, followed by addition of 41 ml of 0.2 N lithium tetrachloropalladate in methanol. After 18 hours stirring at room temperature, the reaction is gravity filtered to remove the black solid palladium, and the yellow methanolic filtrate is treated with five 200 mg portions of sodium borohydride, then concentrated under reduced pressure to solid residue. The residue is purified by flash column chromatography on silica gel eluting 10 vol% methanol in chloroform. Appropriately pure fractions of product are combined and concentrated under reduced pressure to give crystalline 5-[N-(7-trifluoroacetylaminoheptyl)-1-acrylamido]-2'-deoxyuridine (2.8 g). UV λₘₐₓ 302 nm (ε 18000), λₘᵢₙ 230 nm, 280 nm; TLC (silica eluting 15 vol% methanol in chloroform) R_{f} = 0.3.

### Example III

This example illustrates masking of 5'-hydroxyl to produce 5'-dimethoxytrityl-5-(3-trifluoroacetylaminopropenyl)-2'-deoxyuridine as illustrated in Reaction 2.

5-(3-trifluoroacetylaminopropenyl)-2'-deoxyuridine (2.4 g) is thoroughly evaporated twice from pyridine, then stirred in 40 ml pyridine. Dimethoxytrityl (DMT) chloride (2.3 g, 6.6 mmol) is added, and the mixture stirred at room temperature for four hours. After thin layer chromatography (TLC) on silica eluting 10 vol% methanol in chloroform indicates reaction is complete, the reaction is concentrated to a solid residue. This residue is purified by column chromatography on silica eluting chloroform until all faster running impurities have eluted, then bringing off product with 5 vol% methanol in chloroform. The residue is then concentrated to give 5'-dimethoxytrityl-5-(3-trifluoroacetylaminopropen-1-yl)-2'-deoxyuridine as a white fluffy solid (4 g). Product decomposes upon heating; UV λₘₐₓ 291 nm, λₘᵢₙ 266 nm; TLC R_{f} 0.6 on silica eluting 10 vol% methanol in chloroform.

### Example IV

This example illustrates hydrogenation of exocyclic double bond and 5'-hydroxyl masking to produce 5'-dimethoxytrityl-5-(3-trifluoroacetylaminopropyl)-2'-deoxyuridine.

Repeating the nucleoside precursor synthesis and 5'-hydroxyl masking procedures of Examples I and III, but, prior to the addition of the DMT chloride, subjecting the purified 5-(3-trifluoroacetylaminopropenyl)-2'-deoxyuridine to two atmospheres of hydrogen while stirring at room temperature in methanol over 10% palladium-on-carbon catalyst is productive of 5'-dimethoxytrityl-5-(3-trifluoroacetylaminopropyl)-2'-deoxyuridine.

Examples V to VIII illustrate the synthesis of additional modified uracil nucleosides, and subsequent masking of 5'-hydroxyls as represented by Reaction 2.

### Example V

Repeating the nucleoside precursor synthesis and 5' hydroxyl masking procedures of Examples I and III, but replacing N-allyltrifluoroacetamide with compounds numbered *1* through *8* below is productive of the compounds numbered *1'* through *8'* below, respectively; i.e. substitution of
*1* N-(3-butenyl)trichloroacetamide
*2* N-(5-hexenyl)trifluoroacetamide
*3* N-(2-methyl-2-propenyl)trifluoroacetamide
*4* N-(4-ethenylphenylmethyl)trifluoroacetamide
*5* N-(1-methyl-3-butenyl)trifluoroacetamide
*6* N-(12-trichloroaminododecyl)acrylamide
*7* N-(pertrifluoroacetylpolylysyl)acrylamide
*8* N-(3-trifluoroacetylamidopropyl)acrylamide
is productive of
*1*' 5'-dimethoxytrityl-5-(4-trichloroacetylaminobuten-1-yl)-2'-deoxyuridine
*2'* 5'-dimethoxytrityl-5-(6-trifluoroacetylaminohexen-1-yl)-2'-deoxyuridine
*3'* 5'-dimethoxytrityl-5-(3-trifluoroacetylamino-2-methylpropen-1-yl)-2'-deoxyuridine
*4'* 5'-dimethoxytrityl-5-[2-(4-triftuoroacetylaminomethylphenyl)ethen-1-yl]-2'-deoxyuridine
*5*' 5'-dimethoxyltrityl-5-(4-trifluoroacetylamino-4-methylbuten-1-yl)-2'-deoxyuridine
*6*' 5'-dimethoxytrityl-5-[N-(12-trichloroacetylaminododecyl)-1-acrylamido]-2'-deoxyuridine
*7*' 5'-dimethoxytrityl-5-[N-(pertrifluoroacetylpolylysyl)-1-acrylamido]-2'-deoxyuridine
*8'* 5'-dimethoxytrityl-5-[N-(3-trichloroacetylaminopropyl)-acrylamido]-2'-deoxyuridine

### Example VI

Repeating the 5' hydroxyl masking procedure of Example III but replacing 5-(3-trifluoroacetylaminopropenyl)-2'-deoxyuridine with the 5-substituted-2'-deoxyuridines numbered *9* through *18* below is productive of the products numbered *9'* through *18'* below, respectively; i.e. substituting
*9* 5-(propen-1-yl)-2'-deoxyuridine
*10* 5-(carbmethoxyethyl)-2'-deoxyuridine
*11* 5-(3-carbmethoxylprop-1-yl)-2'-deoxyuridine
*12* 5-(4-carbmethoxy-2-methylbuten-1-yl)-2'-deoxyuridine
*13* 5-(3-cyanopropen-1-yl)-2'-deoxyuridine
*14* 5-(4-cyano-2-methylbuten-1-yl)-2'-deoxyuridine
*15* 5-[2-(4-carbmethoxyphenyl)ethen-1-yl]-2'-deoxyuridine
*16* 5-(4-acetoxybuten-1-yl)-2'-deoxyuridine
*17* 5-(4-acetoxybut-1-yl)-2'-deoxyuridine
*18* 5-[4-(2,4-dinitrophenyl)butyl]-2'-deoxyuridine
is productive of the following 5'-dimethoxytrityl-5-alkyl-2'-deoxyuridines
*9'* 5'-dimethoxytrityl-5-(propen-1-yl)-2'-deoxyuridine
*10*' 5'-dimethoxytrityl-5-(2-carbmethoxyethyl)-2'-deoxyuridine
*11'* 5'-dimethoxytrityl-5-(3-carbmethoxyprop-1-yl)-2'-deoxyuridine
*12'* 5'-dimethoxytrityl-5-(4-carbmethoxy-2-methylbuten-1-yl)-2'-deoxyuridine
*13'* 5'-dimethoxytrityl-5-(3-cyanopropen-1-yl)-2'-deoxyuridine
*14'* 5'-dimethoxytrityl-5-(4-cyano-2-methylbuten-1-yl)-2'-deoxyuridine
*15'* 5'-dimethoxytrityl-5-[2-(4-carbmethoxyphenyl)ethen-1-yl]-2'-deoxyuridine
*16'* 5'-dimethoxytrityl-5-(4-acetoxybuten-1-yl)-2'-deoxyuridine
*17'* 5'-dimethoxytrityl-5-(4-acetoxybut-1-yl)-2'-deoxyuridine
*18'* 5'-dimethoxytrityl-5-[4-(2,4-dinitrophenyl)butyl]-2'-deoxyuridiné

### Example VII

Repeating the nucleoside precursor synthesis and 5'-hydroxyl masking procedures of Examples I-VI, but replacing 5-chloromercuri-2'-deoxyuridine with 5-chloromercuriuridine is productive of the corresponding 5'-dimethoxytrityl-5-substituted uridines.

Examples VIII to XI illustrate the synthesis of modified cytosine nucleosides. Since cytosine nucleosides, as well as adenosine nucleosides, have reactive groups on their bases unlike the uracil nucleosides, such reactive groups are masked to prevent unwanted reactions therewith. These examples illustrate masking of reactive groups on the cytosine base moiety as in Reaction 1, as well as masking of the 5'-hydroxyl as in Reaction 2.

### Example VIII

### 5-(3-trifluoroacetylaminopropenyl)-N⁴-benzoyl-2'-deoxycytidine

Repeating the nucleoside precursor synthesis procedure of Example I, but replacing 5-chloromercuri-2'-deoxyuridine with 5-chloromercuri-2'-deoxycytidine is productive of 5-(3-trifluoroacetylaminopropenyl)-2'-deoxycytidine (UV λₘₐₓ 287 mn). Purified 5-(3-trifiuoroacetylaminopropenyl)-2'-deoxycytidine (1.3g, 4.6 mmol) is stirred in 80 ml anhydrous ethanol, benzoyl anhydride (1.5 g, 7 mmol) is added, and the reaction refluxed. Five additional 1.5 g portions of benzoyl anhydride are added hourly. After the reaction is judged complete by thin layer chromatography [silica plates eluting n-butanol/methanol/conc NH₄OH/H₂O (60:20:1:20)] in 6-10 hours, the reaction is cooled and concentrated under reduced pressure to a semisolid. The solid is triturated with ether three times, decanted and dried. The crude product is crystallized from water to give chromatographically pure N⁴-benzoyl-5-(3-trifluoroacetylaminopropenyl)-2'-deoxycytidine as a white solid. The product decomposes above 120°C; UVₘₐₓ 311 nm.

### Example IX

### 5'-Dimethoxytrityl-5-(3-trifluoroacetylaminopropenyl)-N⁴-benzoyl-2'-deoxycytidine

Repeating the 5'-hydroxyl masking procedure of Example III, but replacing 5-(3-trifluoroacetylaminopropenyl)-2'-deoxyuridine with 5-(3-trifluoroacetylaminopropenyl)-N⁴-benzoyl-2'-deoxycytidine is productive of 5'-dimethoxytrityl-5-(3-trifluoroacetylaminopropenyl)-N⁴-benzoyl-2'-deoxycytidine.

### Example X

Repeating the nucleoside precursor synthesis and 5' hydroxyl masking procedures of Examples VIII and IX, but replacing N-allyltrifluoroacetamide with the N-alkyltrifluoroacetamides of Example V is productive of the corresponding 5'-dimethoxytrityl-5-(trifluoroacetylaminoalkyl)-N⁴-benzoyl-2'-deoxycytidines, viz:
5'-dimethoxytrityl-5-(4-trifluoroacetylaminobuten-7-yl)-N⁴-benzoyl-2'-deoxycytidine
5'-dimethoxytrityl-5-(6-trifluoroacetylaminohexen-1-yl)-N⁴-benzoyl-2'-deoxycytidine
5'-dimethoxytrityl-5-(3-trifluoroacetylamino-2-methylpropen-1-yl)-N⁴-benzoyl-2'-deoxycytidine
5'-dimethoxytrityl-5-[2-(4-trifluoroacetylaminomethylphenyl)ethen-1-yl]-N⁴-benzoyl-2'-deoxycytidine
5'-dimethoxytrityl-5-(4-trifluoroacetylamino-4-methylbuten-1-yl)-N⁴-benzoyl-2'-deoxycytidine
5'-dimethoxytrityl-5-[N-(12-trifluoroacetylaminododecyl)-1-acrylamido]-N⁴-benzoyl-2'-deoxycytidine
5'-dimethoxytrityl-5-[N-(pertrifluoroacetylpolylysyl)-1-acrylamido]-N⁴-benzoyl-2'-deoxycytidine

### Example XI

### Synthesis of 5'-dimethoxytrityl-N⁴-benzoyl-5-(2-carbmethoxyethenyl)-2'-deoxycytidine

5-(2-Carbmethoxyethenyl)-2'-deoxycytidine (0.82 g, 2.6 mmol) is stirred in 50 ml anhydrous ethanol. Benzoic anhydride (500 mg, 2.2 mmol) is added, and the reaction heated to reflux. Five additional 500 mg portions of benzoic anhydride are added hourly. After the reaction is judged complete by thin layer chromatography (usually 6-8 hours) the reaction is cooled, and evaporated under reduced pressure to a yellow semi-solid. Chromatography on silica gel eluting a linear 1:19 to 1:3 methanol/chloroform mixture followed by thorough evaporation of appropriately combined fractions gives N⁴-benzoyl-5-(2-carbmethoxyethenyl)-2'-deoxycytidine as an amorphous white solid. UV λₘₐₓ 296 nm, λₘᵢₙ 270 nm. The solid is dried thoroughly, and dissolved in 20 ml pyridine. Dimethoxytrityl chloride (1.1 eq) is added, and the reaction stirred at ambient temperature for six hours. Concentration to a solid followed by column chromatography on silica gel eluting 10% methanol in chloroform yields 5'-dimethoxytrityl-N⁴-benzoyl-5-(2-carbrnethoxyethenyl)-2'-deoxycytidine as a fluffy off-white solid.

### Example XII

Repeating the nucleoside precursor synthesis procedure of Example XI, but replacing 5-(2-carbmethoxyethenyl)-2'-deoxycytidine with the following compounds numbered *19* through *27* below is productive of the corresponding compounds numbered *19'* through *27*' below, respectively, i.e., substituting:
*19* 5-(2-carbmethoxyethyl)-2'-deoxycytidine
*20* 5-(3-carbmethoxyprop-1-yl)-2'-deoxycytidine
*21* 5-(4-carbmethoxy-2-methylbuten-1-yl)-2'-deoxycytidine
*22* 5-(3-cyanopropen-1-yl)-2'-deoxycytidine
*23* 5-(4-cyano-2-methylbuten-1-yl)-2'-deoxycytidine
*24* 5-[2-(4-carbmethoxyphenyl)ethen-1-yl)-2'-deoxycytidine
*25 5*-(4-acetoxybuten-1-yl)-2'-deoxycytidine
*26* 5-(4-acetoxybut-1-yl)-2'-deoxycytidine
*27* 5-[4-(2,4-dinitrophenyl)butyl]-2'-deoxycytidine
is productive of the following 5'-dimethoxytrityl-N⁴-benzoyl-5-alkyl-2'-deoxycytidines:
*19'* 5'-DMT-N⁴-benzoyl-5-(2-carbmethoxyethen-1-yl)-2'-deoxycytidine
*20'* 5'-DMT-N⁴-benzoyl-5-(3-carbmethoxyprop-1-yl)-2'-deoxycytidine
*21'* 5'-DMT-N⁴-benzoyl-5-(4-carbmethoxy-2-methylbuten-1-yl)-2'-deoxycytidine
*22'* 5'-DMT-N⁴-benzoyl-5-(3-cyanopropen-1-yl)-2'-deoxycytidine
*23'* 5'-DMT-N⁴-benzoyl-5-(4-cyano-2-methylbutan-1-yl)-2'-deoxycytidine
*24'* 5'-DMT-N⁴-benzoyl-5-[2-(4-carbmethoxyphenyl)ethen-1-yl)-2'-deoxycytidine
*25'* 5'-DMT-N⁴-benzoyl-5-(4-acetoxybuten-1-yl)-2'-deoxycytidine
*26'* 5'-DMT-N⁴-benzoyl-5-(4-acetoxybut-1-yl)-2'-deoxycytidine
*27*' 5'-DMT-N⁴-benzoyl-5-[4-(2,4-dinitrophenyl)butyl]-2'-deoxycytidine

Similarly, the use of the other acid anhydrides, e.g., acetic anhydride, anisoyl anhydride, or tolyl anhydride, is productive of the corresponding N⁴-acyl or N⁴-acetyl 5-alkyl-2'-deoxycytidines of Examples X and XI wherein benzoyl is replaced by acetyl or acyl.

### Example XIII

Repeating the nucleoside precursor synthesis and 5'-hydroxyl masking procedures of Examples VIII to X, but replacing 5-chloromercuri-2'-deoxycytidine with 5-chloromercuricytidine is productive of the corresponding 5'-dimethoxytrityl-N⁴-benzoyl-5-substituted cytidines.

### Example XIV

This example typifies the masking of reactive base moieties and the masking of 5' hydroxyl of adenine nucleosides.

N⁶-benzoyl-8-(6-aminohexyl)amino-2'-deoxyadenosine (4 mmol) is stirred in 60 ml anhydrous ethanol. Trifluoroacetic anhydride (6 mmol) is added, and the reaction stirred at room temperature. Two additional portions of trifluoroacetic anhydride are added hourly. After four hours, the reaction is concentrated to a solid residue, and lyophilized overnight. The crude N⁶-benzoyl-8-(6-trifluoroacetylaminohexyl)amino-2'-deoxyadenosine is dried thoroughly and concentrated to a solid residue twice from pyridine. The solid is stirred in 40 ml of pyridine, and dimethoxytrityl chloride (6.5 mmol) is added. After four hours, the reaction is concentrated to leave a solid residue. Purification by column chromatography on silica gel eluting a multi-step gradient of 0 to 15% methanol in chloroform gives 5'-dimethoxytrityl-N⁶-benzoyl-8-(6-trifluoroacetylaminohexyl)amino-2'-deoxyadenosine as an off-white solid.

Examples XV to XVII typify the activation of 5'-masked 5-substituted, and naturally occurring nucleosides, to their respective phosphomonochloridites, as illustrated in Reaction 3 of the diagram.

### Example XV

### Preparation of 3'-phosphomonochloridite of 5'-DMT-5-(3-trifluoroacetylaminoprop-1-yl)-2'-deoxyuridine

Dry 5'-DMT-5-(3-trifluoroacetylaminoprop-1-yl)-2'-deoxyuridine (1.54 g, 2.2 mmol) is lyophilized from 20 ml benzene three times for more than twelve hours each to remove residual water and solvents. The resulting very fluffy white powder is transferred to a nitrogen atmosphere while still under vacuum and dissolved in anhydrous acetonitrile containing 5 vol% 2,6-lutidine to a final nucleoside concentration of 30 mM. While swirling vigorously under nitrogen, one rapid bolus of methylphosphodichloridite (1.0 eq) is added by syringe. The reaction is swirled for about one minute under nitrogen. The resulting crude 5'-DMT-5-(3-trifluoroacetylaminoprop-1-yl)-2'-deoxyuridine 3'-methylphosphomonochloridite reaction solution is then used directly for deoxyoligonucleotide synthesis (Example XVIII) with no further purification, [³¹P-NMR(CH₃CN/CDCl₃) generally indicates 40-70 mol% desired product (167.5 ppm); remainder is composed of *bis*-3',3'-[5'-DMT-5-(3-trifluoroacetylaminoprop-1-yl]-2'-deoxyuridylyl] methylphosphite (140 ppm) and 5'-DMT-5-(3-trlfluoroacetylaminoprop-1-yl)-2'-deoxyuridine 3'-methylphosphonate (9.5 ppm), the latter product being formed in amounts reflecting the presence of water in the reaction].

### Example XVI

### Preparation of 3'-phosphomonochloridites of the naturally-occurring 2'-deoxynucleosides

Repeating the procedure of Example XV, but replacing 5'-DMT-5-(3-trifluoroacetylaminoprop-1-yl)-2'-deoxyuridine with:
5'-DMT-2'-deoxythymidine
5'-DMT-N⁴-benzoyl-2'-deoxycytidine
5'-DMT-N⁶-benzoyl-2'-deoxyadenosine
5'-DMT-N²-isobutyryl-2'-deoxyguanosine
is productive of the corresponding phosphomonochloridites, viz.:
5'-DMT-2'-deoxythymidine 3'-methylphosphomonochloridite
5'-DMT-N⁴-benzoyl-2'-deoxycytidine 3'-methylphosphomonochloridite
5'-DMT-N⁶-benzoyl-2'-deoxyadenosine 3'-methylphosphomonochloridite
5'-DMT-N²-isobutyryl-2'-deoxyguanosine 3'-methylphosphomonochloridite

### Example XVII

Repeating the phosphomonochloridite synthesis procedures of Examples XV and XVI, but replacing methylphosphodichloridite with *o*-chlorophenylphosphodichloridite is productive of the corresponding 5'-DMT-nucleoside 3'-phosphomonochloridites, viz.:
5'-DMT-5-(3-trifluoroacetylaminopropyl)-2'-deoxyuridine 3'-*o*-chlorophenylphosphomonochloridite
5'-DMT-2'-deoxythymidine 3'-*o*-chlorophenylphosphomonochloridite
5'-DMT-N⁴-benzoyl-2'-deoxycytidine 3'-*o*-chlorophenylphosphomonochloridite
5'-DMT-N⁶-benzoyl-2'-deoxyadenosine 3'-*o*-chlorophenylphosphomonochloridite
5'-DMT-N²-isobutyryl-2'-deoxyguanosine 3'-*o*-chlorophenylphosphomonochloridite

Similarly, the use of p-chlorophenylphosphodichloridite is productive of the analogous 3'-*p*-chlorophenylphosphomonochloridite adducts. [³²P]NMR (CH₃CN CDCl₃) of o-chlorophenylphosphomonochloridite products 160.7, 160.5 ppm (diasteriomers).

Examples XVIII-XXIV typify the chemical synthesis of oligonucleotides which incorporate modified bases, as illustrated by Reactions 4 and 5 in the diagram.

### Example XVIII

### Synthesis of deoxyoligonucleotides containing 5-(3-aminopropyl)-uracil and naturally-occurring nucleotide units

The phosphomonochloridite synthesis procedures of Examples XV and XVI are accomplished immediately before deoxyoligonucleotide synthesis, and the resulting products are used directly as 30 mM crude 3'-methylphosphomonochloridites in anhydrous acetonitrile/5 vol% 2,6-lutidine.

Solid support (5-DMT-N⁶-benzoyl-2'-deoxyadenosine 3'-succinamidepropyl silica, 250 mg, 20 µeq) is put into a suitable reaction flow vessel (glass or Teflon® column or funnel). The solid support is preconditioned by successive treatments with acetonitrile/5 vol% lutidine, 2 w/v% iodine in tetrahydro-furan/water/lutidine for 2 minutes, acetonitrile/5% lutidine, chloroform, 4 vol% dichloroacetic acid in chloroform for 2.5 minutes, and acetonitrile/5% lutidine, where treatments are total volumes of 5-15 ml in either 2 or 3 portions or by constant flow, as desired.

The deoxyoligonucleotide is synthesized in accordance with Reaction 4 by sequential addition of the desired activated 5'-DMT-nucleoside 3'-methylphosphomonochloridite monomer and coupling thereof to the free 5'-hydroxyl of the terminal unit of the growing nucleotide chain, which unit is initially the only unit of the chain, i.e., the deoxyadenosine based unit comprising the solid support. Additions are by reaction of 10 ml of the crude 30 mM monochloridites chosen from Examples XV and XVI with the now-unprotected 5' hydroxyl of the chain in either 2 or 3 portions or by constant flow, for 2 to 6 minutes. The first phosphomonochloridite addition followed by one complete reagent cycle consists of sequential treatments with:
5'-DMT-5-(3-trifluoroacetylaminopropyl)-2'-deoxyuridine 3'-methylphosphomonochloridite acetonitrile/lutidine wash
capping for 5 minutes with 0.3 M 4-dimethylaminopyridine in acetic anhydride/lutidine/tetrahydrofuran (1:3:2)
acetonitrile/5% lutidine wash
oxidation with 2% iodine in tetrahydrofuran/water/lutidine (6:2:1) for 2 minutes
acetonitrile/5% lutidine wash
chloroform wash
removal of DMT by 2.5 minute treatment with 4 vol% dichloroacetic acid in chloroform
chloroform wash
acetonitrile/lutidine wash

The above cycle is repeated thirteen times, each time replacing 5'-DMT-5-(3-trifluoroacetylaminopropyl)-2'-deoxyuridine 3'-methylphosphomonochloridite with a different one of the following 3'-methylphosphomonochloridites:
5'-DMT-2',-deoxythymidine 3'-methylphosphomonochloridite
5'-DMT-5-(3-trifluoroacetylaminopropyl)-2'-deoxyuridine 3'-methylphosphomonochloridite
5'-DMT-N⁶-benzoyl-2'-deoxyadenosine 3'-methylphosphomonochloridite
5'-DMT-N⁴-benzoyl-2'-deoxycytidine 3'-methylphosphomonochloridite
5'-DMT-N²-isobutyryl-2'-deoxyguanosine 3'-methylphosphomonochloridite
5'-DMT-5-(3-trifluoroacetylaminopropyl)-2'-deoxyuridine 3'-methylphosphomonochloridite
5'-DMT-2'-deoxythymidine 3'-methylphosphomonochloridite
5'-DMT-5-(3-trifluoroacetylaminopropyl)-2'-deoxyuridine 3'-methylphosphomonochloridite
5'-DMT-deoxythmidine 3'-methylphosphomonochloridite
5'-DMT-5-(3-trifluoroacetylaminopropyl)-2'-deoxyuridine 3'-methylphosphomonochloridite
5'-DMT-N²-isobutyryl-2'-deoxyguanosine 3'-methylphosphomonochloridite
5'-DMT-N⁶-benzoyl-2'-deoxyadenosine 3'-methylphosphomonochloridite
5'-DMT-N⁴-benzoyl-2'-deoxycytidine 3'-methylphosphomonochloridite
in respective order, deleting dichloroacetic acid treatment during the last reagent cycle. The support is transferred and treated with 2 ml concentrated ammonium hydroxide for 4 hours at ambient temperature to release the product from the support. The supernatant is removed, the solid washed three times with 0.5 ml concentrated ammonium hydroxide, and the combined supernatants are sealed and heated at 50°C overnight. The clear yellow supernatant is lyophilized thoroughly. Initial purification is accomplished by reverse phase high pressure liquid chromatography (HPLC) on an RP―8 (C―8) column eluting a 60 minute linear gradient of 0 to 30% vol% acetonitrile in 25 mM ammonium acetate, pH 6.8. The 5'-DMT-terminated product, eluting as a sharp peak at about 40 minutes, is collected; all shorter chains, both capped and uncapped, elute before 25 minutes. The collected product is evaporated to a solid residue, treated with 80% acetic acid at ambient temperature for 20 minutes (to remove DMT), lyophilized to a solid residue, and dissolved in a small amount of aqueous buffer. The product, generally greater than 90% homogeneous after HPLC, is further purified by conventional electrophoresis on 20% polyacrylamide gels (1 to 6 mm thick) by excision and extraction of the appropriate product band (product generally migrates slower than unmodified deoxyoligonucleodites of similar length). The purified 5-aminopropyl-uracil-containing pentadecadeoxyoligonucleotide product illustrated diagrammatically below, wherein U^{m} = 5-(3 aminopropyl)uracil, is thereby produced.

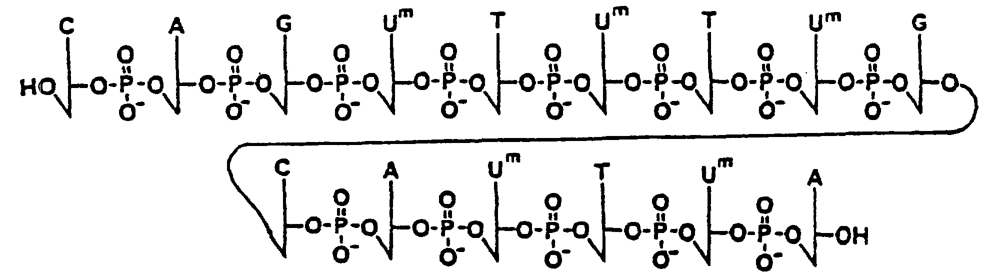

Note the conventional deprotection of the oligonucleotide with ammonia has also removed the trifluoroacetyl masking group on the substituent

The length and sequence of this oligonucleotide may then be determined by ³²P-kinasing and sequencing using suitable protocols, for example the protocols heretofore used to determine the length and sequence of the prior art oligonucleotides in which the bases of the nucleotide units therein are unmodified.

Similarly, intentional variation of the order and number of methylphosphomonochloridite additions employed here is productive of other 5-(modified)uracil-containing deoxyoligonucleotides which vary in selected length and base sequence. In addition, replacement of the nucleoside 3'-methylphosphomonochloridite adducts of Examples XV and XVI with the corresponding 3'-*o*- or *p*-chlorophenylphosphomonochloridite adducts of Example XVII and inclusion of pyridinium oximate treatment to remove chlorophenyl blocking groups (at the end of the deoxyoligonucleotide synthesis and before concentrated ammonium hydroxide treatment) is productive of the same deoxyoligonucleotide products.

### Example XIX

Repeating the phosphomonochloridite and deoxyoligonucleotide synthesis procedures of Examples XV to XVIII, but replacing 5'-DMT-5-(3-trifluoroacetylaminopropyl)-2'-deoxyuridine with the 5'-DMT-5-alkyl-2'-deoxyuridines numbered 28 through 36 below is productive of the corresponding oligonucleotides having uracil bases U^{m} numbered *28'* through 36' below, respectively, i.e., substituting:
*28* 5'-dimethoxytrityl-5-(3-trifluoroacetylaminopropen-1-yl)-deoxyuridine
*29* 5'-dimethoxytrityl-5-(4-trifluoroacetylaminobut-1-yl)-2'-deoxyuridine
*30* 5'-dimethoxytrityl-5-(4-trifluoroacetylaminobuten-1-yl)-2'-deoxyuridine
*31* 5'-dimethoxytrityl-5-(6-trifluoroacetylaminohex-1-yl)-2'-deoxyuridine
*32* 5'-dimethoxytrityl-5-(6-trifluoroacetylaminohexen-1-yl)-2'-deoxyuridine
*33* 5'-dimethoxytrityl-5-(2-trifluoroacetylaminoprop-2-yl)-2'-deoxyuridine
*34* 5'-dimethoxytrityl-5-(3-trifluoroacetylamino-2-methyl-propen-1-yl)-2'-deoxyuridine
*35* 5'-dimethoxytrityl-5-(3-trifluoroacetylamino-2-methyl-prop-1-yl)-2'-deoxyuridine
*36* 5'-dimethoxytrityl-5-[2-(4-trifluoroacetylaminomethylphenyl)ethen-1-yl]-2'-deoxyuridine
*37* 5'-dimethoxytrityl-5-[N-(pertrifluoroacetylpolylysyl)-1-acrylamidol-2'-deoxyuridine
*38* 5'-DMT-5-[N-(7-trifluoroacetylaminoheptyl)-1-acrylamido]-2'-deoxyuridine
is productive of the deoxynucteotides corresponding to the product of Example XVIII, wherein U^{m} is:
*28'* 5-(3-aminopropen-1-yl) uracil
*29'* 5-(4-aminobut-1-yl) uracil
*30'* 5-(4-aminobuten-1-yl) uracil
*31'* 5-(6-aminohex-1-yl) uracil
*32'* 5-(6-aminohexen-1-yl) uracil
*33'* 5-(3-aminoprop-2-yl) uracil
*34'* 5-(3-amino-2-methylpropen-1-yl) uracil
*35'* 5-(3-amino-2-methylprop-1-yl) uracil
*36'* 5-[2-(4-aminoethylphenyl)ethen-1-yl] uracil
*37'* 5-[N-(polylysyl)-1-acrylamidol uracil
*38'* 5-[N-(7-aminoheptyl)-1-acrylamido] uracil

Similarly, by employing other 5'-DMT-5-(acylaminoalkyl)-2'-deoxyuridines, the analogous deoxyoligonucleotides are produced.

### Example XX

Repeating the phosphomonochloridite and deoxyoligonucleotide synthesis procedures of Examples XV to XVIII, but replacing 5'-DMT-5-(3-acetylaminopropyl)-2'-deoxyuridine with 5-substituted-2'-deoxyuridines numbered *37* through *46* below is productive of the corresponding oligonucleotides having the U^{m} uracil bases numbered *37'* through *46'*, below respectively; i.e., substituting:
*37* 5'-DMT-5-(propen-1-yl)-2'-deoxyuridine
*32* 5'-DMT-5-(2-carbmethoxyethyl)-2'-deoxyuridine
*39* 5'-DMT-5-(3-cerbmethoxyprop-1-yl)-2'-deoxyuridine
*40* 5'-DMT-5-(4-carbmethoxy-2-methylbuten-1-yl)-2'-deoxyuridine
*41* 5'-DMT-5-(3-cyanopropen-1-yl)-2'-deoxyuridine
*42* 5'-DMT-5-(4-cyano-2-methylbuten-1-yl)-2'-deoxyuridine
*43* 5'-DMT-5-[2-(4-carbmethoxyphenyl)ethen-1-yl]-2'-deoxyuridine
*44* 5'-DMT-5-(4-acetoxybuten-1-yl)-2'-deoxyuridine
*45* 5'-DMT-5-(4-acetoxybut-1-yl)-2'-deoxyuridine
*46* 5'-DMT-5-[4-(2,4-dinitrophenyl)butyl)-2'-deoxyuridine
is productive of the products wherein, U^{m} is:
*37*' 5-(propen-1-yl)uracil
*38'* 5-(2-carboxyethyl)uracil
*39'* 5-(3-carboxyprop-1-yl)uracil
*40'* 5-(4-carboxy-2-methylbuten-1-yl)uracil
*41'* 5-(3-cyanopropen-1-yl)uracil
*42'* 5-(4-cyano-2-methylbuten-1-yl)uracil
*43'* 5-[2-(4-carboxyphenyl)ethen-1-yl]uracil
*44'* 5-(4-hydroxybuten-1-yl)uracil
*45'* 5-(4-hydroxbut-1-yl)uracil
*46'* 5-[4-(2,4-dinitrophenyl)butyl]uracil

Note: In *46*' the dinitrophenyl is a ligand recognition type reporter group, i.e. use of antidinitrophenyl antibody as the ligand. Similarly, by employing other appropriate 5'-DMT-5-alkyl-2'-deoxyuridines, the analogous deoxyoligonucleotides are produced.

### Example XXI

Repeating the procedures of Examples XV to XVIII, but replacing 5'-DMT-5-(3-trifluoroacetylaminopropyl)-2'-deoxyuridine with 5'-DMT-N⁴-benzoyl-5-(3-trichloroacetylaminopropyl)-2'-deoxycytidine is productive of deoxyoligonucleotides as in Example XVIII wherein U^{m} [5-(3-aminopropyl)-uracil] is replaced by 5-(3-aminopropyl)cytosines. For example, 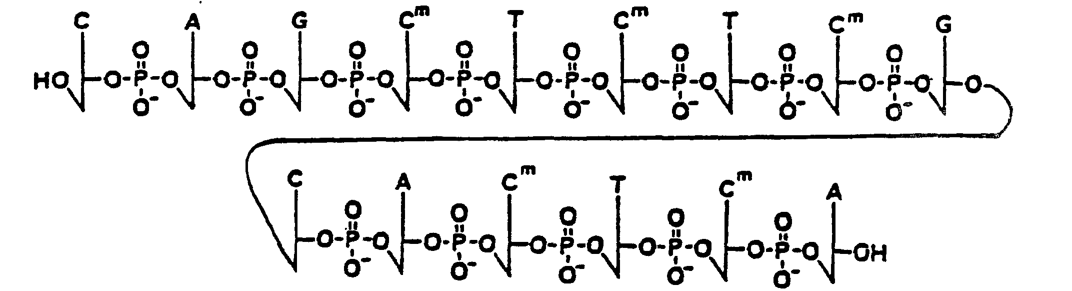 where C^{m} = 5-(3-aminopropyl)cytosine.

### Example XXII

Repeating the deoxyoligonucleotide synthesis procedure of Example XXI but replacing 5'-DMT-N⁴-benzoyl-5-(3-trichloroacetylaminopropyl)-2'-deoxycytidine with compounds numbered *47* through *57* below is productive of the corresponding oligonucleotides having the C^{m} cytosine bases numbered *47'* through *57*' below, respectively, i.e., substituting:
*47* 5-OMT-N⁴-benzoyl-5-(3-trifluorcacetylaminopropen-1-yl)-2'-deoxycytidine
*48* 5'-DMT-N⁴-benzoyl-5-(4-trifluoroacetylaminobut-1-yl)-2'-deoxycytidine
*49* 5'-DMT-N⁴-benzoyl-5-(4-trifluoroacetylaminobuten-1-yl)-2'-deoxycytidine
*50* 5'-DMT-N⁴-benzoyl-5-(6-trifluoroacetylaminohex-1-yl)-2'-deoxycytidine
*51* 5'-DMT-N⁴-benzoyl-5-(6-trifluoroacetylaminohexen-1-yl)-2'-deoxycytidine
*52* 5'-DMT-N⁴-benzoyl-5-(3-trifluoroacetylaminoprop-2-yl)-2'-deoxycytidine
*53* 5'-DMT-N⁴-benzoyl-5-(3-trifluoroacetylamino-2-methylpropen-1-yl)-2'-deoxycytidine
*54* 5'-DMT-N⁴-benzoyl-5-(3-trifluoroacetylamino-2-methylprop-1-yl)-2'-deoxycytidine
*55* 5'-DMT-N⁴-benzoyl-5-[2-(4-trifluoroacetylaminomethylphenyl)ethen-1-yl]-2'-deoxycytidine
*56* 5'-DMT-N⁴-benzoyl-5-[N-(pertrifluoroacetylpolylysyl)-1-acrylamido]-2'-deoxycytidine
*57* 5'-DMT-N⁴-benzoyl-5-[N-(trifluoroacetylaminoheptyl)-acrylamido]-2'-deoxycytidine
is productive of the products wherein C^{m} is:
*47'* 5-(3-aminopropen-1-yl)cytosine
*48'* 5-(4-aminobut-1-yl)cytosine
*49'* 5-(4-aminobuten-1-yl)cytosine
*50'* 5-(6-aminohex-1-yl)cytosine
*51'* 5-(6-aminohexen-1-yl)cytosine
*52'* 5-(3-aminoprop-2-yl)cytosine
*53'* 5-(3-amino-2-methylpropen-1-yl)cytosine
*54'* 5-(3-amino-2-methylprop-1-yl)cytosine
*55'* 5-[2-(4-aminomethylphenyl)ethen-1-yl)cytosine
*56'* 5-[N-(polylysyl)-1-acrylamido]cytosine
*57*' 5-[N-(7-aminoheptyl)-1-acrylamido]cytosine

Similarly, by employing other N⁴-acyl-5-(acylaminoalkyl)-2'-deoxycytidines the analogous deoxyoligonucleotides are produced

### Example XXIII

Repeating the deoxyoligonucleotide synthesis procedure of Example XXI, but replacing 5'-DMT-N⁴-benzoyl-5-(3-trifluoroacetylaminopropyl)-2'-deoxycytidine with the compounds numbered *58* through *68* below is productive of the corresponding oligonucleotides having the C^{m} cytosine bases numbered *58'* through *68'* below, respectively, i.e., substituting:
*58* 5'-DMT-N⁴-benzoyl-5-(propen-1-yl)-2'-deoxycytidine
*59* 5'-DMT-N⁴-benzoyl-5-(2-carbmethoxyethyl)-2' deoxycytidine
*60* 5'-DMT-N⁴-benzoyl-5-(2-carbmethoxyethen-1-yl)-2'-deoxycytidine
*61* 5'-DMT-N⁴-benzoyl-5-(3-carbmethoxyprop-1-yl)-2'-deoxycytidine
*62* 5'-DMT-N⁴-benzoyl-5-(4-carbmethoxy-2-methylbuten-1-yl)-2'-deoxycytidine
*63* 5'-DMT-N⁴-benzoyl-5-(3-cyanopropen-1-yl)-2'-deoxycytidine
*64* 5'-DMT-N⁴-benzoyl-5-(4-cyano-2-methylbuten-1-yl)-2'-deoxycytidine
*65* 5'-DMT-N⁴-benzoyl-5-[2-(4-carbmethoxyphenyl)ethen-1-yl]-2'-deoxycytidine
*66* 5'-DMT-N⁴-benzoyl-5-(4-acetoxybuten-1-yl)-2'-deoxycytidine
*67* 5'-DMT-N⁴-benzoyl-5-(4-acetoxybut-1-yl)-2'-deoxycytidine
*68* 5'-DMT-N⁴-benzoyl-5-[4-(2,4-dinitrophenyl)butyl]-2-deoxycytidine
is productive of the products wherein C^{m} is:
*58'* 5-(propen-1-yl)cytosine
*59*' 5-(2-carboxyethyl)cytosine
*60'* 5-(2-carboxyethen-1-yl)cytosine
*61'* 5-(3-carboxyprop-1-yl)cytosine
*62*' 5-(4-carboxy-2-methylbuten-1-yl)cytosine
*63*' 5-(3-cyanopropen-1-yl)cytosine
*64*' 5-(4-cyano-2-methylbuten-1-yl)cytosine
*65'* 5-[2-(4-carboxyphenyl)ethen-1-yl]cytosine
*66'* 5-(4-hydroxybuten-1-yl)cytosine
*67'* 5-(4-hydroxybut-1-yl)cytosine
*68'* 5-[4-(2,4-dinitrophenyl)butyl]cytosine

Similarly, by employing other appropriate 5'-DMT-N⁴-acyl-5-alkyl-2'-deoxycytidines the analogous deoxyoligonucleotides are produced

### Example XXIV

Repeating the phosphomonochloridite and deoxyoligonucleotide synthesis procedures of Examples XV-XXIII, but replacing 5'-DMT-5-(3-trifluoroacetylaminopropyl)-2'-deoxyuridine with 5'-DMT-N⁶-benzoyl-8-(6-trifluoroacetylaminohexyl)amino-2'-deoxyadenosine is productive of deoxyoligonucleotides as in Example XVIII, except that the U^{m} is replaced by A^{m}, and A^{m} = 8-(6-aminohexyl) amino-2'-deoxyadenosine.

Examples XXV to XXVIII typify the binding of reporter groups to oligonucleotides containing appropriately modified bases, as illustrated in Reaction 6.

### Example XXV

### Fluoresceinated deoxyoligonucleotides

A purified pentadeca-nucleotide (from Example XVIII) of the structure 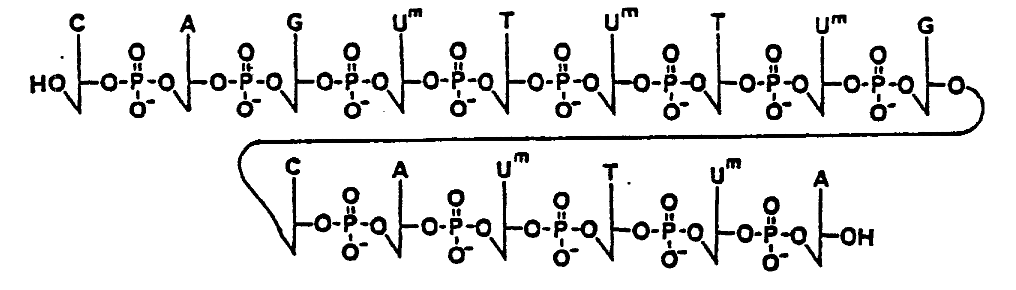 [where U^{m} is 5-[N-(7-aminoheptyl)-1-acrylamido]uracil is dissolved at 25 A₂₆₀ units per ml in aqueous 300 mM sodium borate or sodium carbonate buffer, pH 9.5, containing 30 mM sodium chloride. Solid fluorescein isothiocyanate (0.5 mg per ml) is added, and the mixture sealed and shaken gently at 4°C to 25°C overnight. The reaction is chromatographed directly on a column of G-50 Sephadex® to separate unbound fluorescein adducts which are retained; the fluoresceinated deoxyoligonucleotide adducts elute near the void volume. Early fractions containing significant A₂₆₀ units are combined and lyophilized to solid product of structure similar to the starting pentadecadeoxyoligonucleotide where U^{m} is now either 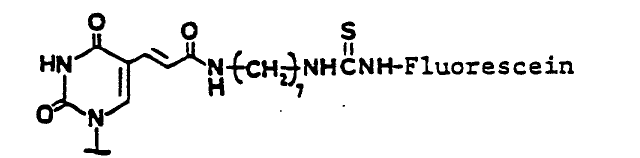 or unreacted 5-[N-(7-aminoheptyl)-1-acrylamido)uracil. λₘₐₓ (H₂O) 262 nm, 498 nm.

Repeating the procedure on compounds recited in Examples XIX, XXI, XXII, and XXIV is productive of the corresponding fluoresceinated or polyfluoresceinated deoxyoligonucleotides in like manner.

### Example XXVI

Attachment of reporter groups other than fluorescein can be accomplished by repeating the procedure of Example XXV, but replacing fluorescein isothiocyanate with, for example:
2,4-dinitrophenyl isothiocyanate
1-fluoro-2,4-dinitrobenzene
aminoethyl isoluminol isothiocyanate
aminoethylaminonaphthalene-1,2-carboxylic hydrazide isothiocyanate
N,N'-*bis* (alkylsulfonyl)-N-aryl-N'-isothiocyanatoaryldioxamide
*m*-sulfonyl aniline isothiocyanate
N-hydroxysuccinimidyl biotin
9-(N-hydroxysuccinimidyl carboxy)-N-methylacridine, or cyanogenbromide-activated Sepharose®
is productive of the corresponding adducts wherein the attached group is other than fluorescein.

### Example XXVII

### Attachment of isoluminol and free primary amine-containing reporter groups

A purified pentadecanucleotide from Example XVIII of the structure: 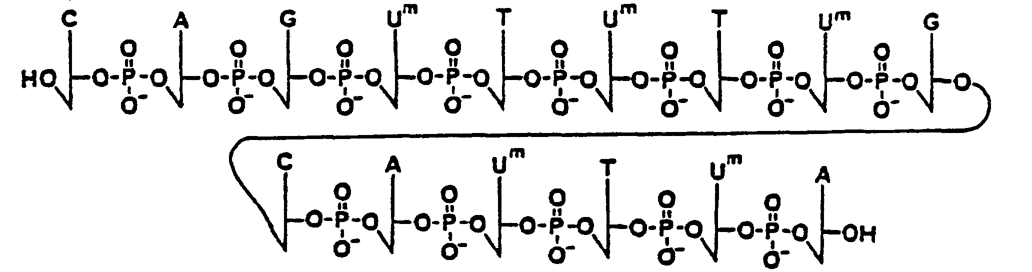 [where U^{m} is 5-(2-carboxyethenyl)uracil] is dissolved in water at 30 A₂₆₀ units per ml., and diluted with one volume pyridine. Aminobutyl ethyl isoluminol is added to a final concentration of 1 mg/ml, followed by addition of a five-fold molar excess of 1-ethyl-3-(3-dimethylaminopropyl) carbodiimide. The reaction is sealed and shaken gently in the dark for 12 to 48 hours. The reaction mixture is concentrated under reduced pressure to a solid residue, and chromatographed directly on a column of G-50 Sephadex®; the isoluminoldeoxyoligonucleotide conjugates elute near the void volume. Early fractions containing significant A₂₆₀ units are combined and lyophilized to solid product of structure similar to the starting deoxyoligonucleotide where U^{m} is now either 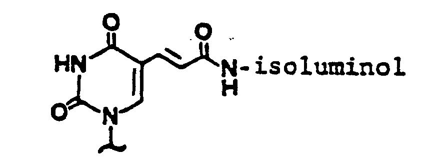 or unreacted 5-(2-carboxyethenyl)uracil.

Repeating the procedure on compounds from Examples XV and XXIII wherein R₂ contains carboxy is productive of the corrresponding deoxyoligonucleotide-isoluminol adducts in like manner.

Repeating the procedure, but replacing aminobutyl isoluminol with other reporter groups containing a free primary amine is productive of the corresponding deoxyoligonucleotide-reporter adducts in like manner.

### Example XXVIII

### Attachment of dinitrophenyl reporter groups

A purified nonanucleotide of the structure: 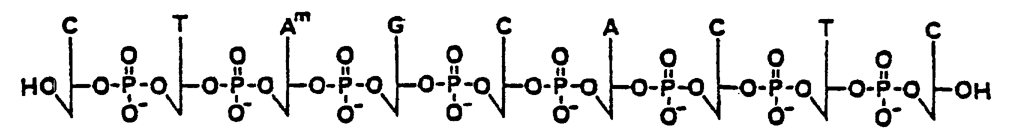 where A^{m} = 8-(6-aminohexyl)aminoadenine is dissolved at 20 A₂₆₀ units per ml in 250 mM sodium carbonate buffer, pH 9, and 1-fluoro-2,4-dinitrobenzene is added. The reaction solution is shaken at ambient temperature overnight, then chromatographed directly on a column of Sephadex® G―50. Early fractions containing significant A₂₆₀ units are combined and concentrated to give an oligonucleotide product similar to the starting decanucleotide wherein A^{m} is now either 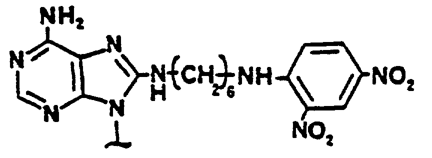 or unreacted 8-(6-aminohexyl)aminoadenine.

Repeating the procedure, but replacing 8-(6-aminohexyl)aminoadenine with other modified bases containing a free primary amine is similarly productive of the corresponding dinitrophenylated oligonucleotide adducts.

## Claims

1. A homogeneous defined sequence single strand oligonucleotide less than 0.2 kb in length incorporating at least one ribonucleotide or deoxyribonucleotide unit having a pyrimidine or purine base modified at a sterically tolerant site, by attachment thereto of a substituent group which has bound thereto at least one non-radioisotopic reporter group or solid support, the substituent group, when the modified base is a pyrimidine, incorporating a chain of two or more carbon atoms, and when the modified base is a purine, incorporating a chain of one or more carbon atoms attached to the base through a polyvalent heteroatom, each such modified base being located at a preselected position in the oligonucleotide.

2. The oligonucleotide of claim 1 wherein the substituent is attached at the C-5 position of a pyrimidine base or at the C-8 position of a purine base.

3. The oligonucleotide of claim 1 or claim 2 wherein the substituent group is bound to a solid support or has bound thereto at least one functionally colorimetric, fluorescent, luminescent, or ligand recognition type non-radioisotopic reporter group.

4. The oligonucleotide of any preceding claim wherein said modified base is a pyrimidine, and the reporter group or solid support has been bound by the substituent group having at least one terminal amine group attached to said carbon chain remote from the C-5 position.

5. The oligonucleotide of claim 4 wherein a solid support or a functionally colorimetric, fluorescent, luminescent, or ligand recognition type non-radioisotopic reporter group, has been attached to said amine group.

6. The oligonucleotide of any one of claims 1 to 3 wherein said modified base is uracil or cytosine and said substituent group has been -CH=CR₁CₙH₂ₙY, -CH₂CHR₁CₙH₂ₙY, 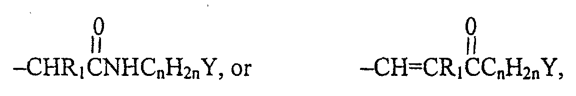 wherein R₁ is hydrogen or C₁₋₆ lower alkyl, n is 0 to 20, and Y is at least one amine or masked amine, carboxy or masked carboxy, hydroxy or masked hydroxy, carboxyphenyl or masked carboxyphenyl, aminoalkylphenyl or masked aminoalkylphenyl, or an adduct thereof.

7. The oligonucleotide of any one of claims 1 to 3 wherein said modified base is adenine and said substituent group has been -CHR₁CₙH₂ₙY and has been attached to the C-8 position of said adenine base through a nitrogen, oxygen, or sulfur atom, R₁ is hydrogen or C₁₋₆ lower alkyl, n is 0 to 20, and Y is at least one amine or masked amine, hydroxy or masked hydroxy, carboxy or masked carboxy, carboxyphenyl or masked carboxyphenyl, aminoalkylphenyl, or masked aminoalkylphenyl, or an adduct thereof.

8. The oligonucleotide of claim 6 or claim 7 wherein n is 3 to 12.

9. The oligonucleotide of any one claims 6 to 8 wherein at least one functionally colorimetric, luminescent, fluorescent, or ligand recognition type non-radioisotopic reporter group is bound to Y.

10. The oligonucleotide of any preceding claim wherein said at least one non-radioisotopic reporter group is fluorescein, fluorescamine, rhodamine, an acridinium salt, nitrophenyl, dinitrophenyl, benzenesulfonyl, biotin, iminobiotin, desthiobiotin, luminol, isoluminol, luciferin, dioxetane, dioxamide, or an adduct thereof.

11. The oligonucleotide of claim 10 wherein the substituent group is attached to the adenine base through a nitrogen atom, and at least one reporter group is bound to Y, which reporter group is fluorescein, fluorescamine, rhodamine, an acridinium salt, nitrophenyl, dinitrophenyl, benzenesulfonyl, luminol, isoluminol, luciferin, dioxetane, dioxamide, biotin, iminobiotin, desthiobiotin or an adduct thereof.

12. The oligonucleotide of any one of claims 6 to 8 which has been attached to a solid support through Y.

13. The oligonucleotide of any one of the preceding claims which is from about 5 to about 60 base units in length.

14. The oligonucleotide of claim 13 which is from about 10 to about 40 base units in length.

15. A process for the chemical synthesis of a defined sequence single strand oligonucleotide incorporating at least one modified base, comprising condensing to an unprotected hydroxyl-bearing terminal unit of a nucleotide chain at a preselected position, a nucleotide monomer having attached thereto an activated phosphorus-containing group, whereby said chain is extended by coupling of said monomer thereto through said active phosphorus-containing group at the site of said unprotected hydroxyl, said nucleotide chain having fewer than 0.2 kb, and at least one of said monomer and terminal units having a ribonucleotide or deoxyribonucleotide pyrimidine or purine base modified at a sterically tolerant site by attachment thereto of a substituent, the substituent group, when the modified base is a pyrimidine, incorporating a chain of two or more carbon atoms, and when the modified base is a purine, incorporating a chain of one or more carbon atoms attached to the base through a polyvalent heteroatom, wherein prior to or after the coupling step at least one non-radioisotopic reporter group or solid support is bound to said substituent, said substituent group during said coupling step having no reporter group bound thereto or having a masked or unmasked reporter group bound thereto which has no substantial adverse effect on said condensation reaction.

16. The process of claim 15 wherein the ribonucleotide or deoxyribonucleotide base is modified at the C-5 position of a pyrimidine base and at the C-8 position of a purine base.

17. The process of claim 15 or claim 16 wherein said phosphorus-containing group is at the 3' or 5' position of the nucleotide monomer and said unprotected hydroxyl of the terminal unit is at the 5' position of the latter when the phosphorus-containing group is at the 3' position of the monomer and is at the 3' position of the terminal unit when the phosphorus-containing group is at the 5' position of the monomer.

18. The process of any one of claims 15 to 17 wherein, prior to or after the coupling step, at least one of said monomer and nucleotide chain is attached to a solid support.

19. The process of any one of claims 15 to 17 wherein, prior to or after the coupling step, at least one functionally colorimetric, fluorescent, luminescent or ligand recognition type non-radioisotopic reporter group is bound to said substituent group, said reporter group, when bound prior to the coupling step, being in masked form if it would otherwise adversely affect the coupling reaction.

20. The process of claim 19 wherein said at least one non-radioisotopic reporter group is bound to said substituent group after the coupling step and is fluorescein, fluorescamine, rhodamine, an acridinium salt, nitrophenyl, dinitrophenyl, benzenesulfonyl, biotin, iminobiotin, desthiobiotin, luminol, isoluminol, luciferin, dioxetane, dioxamide, or an adduct thereof.

21. The process of any one of claims 15 to 20 wherein said modified base is uracil or cytosine and said substituent group is -CH=CR₁CₙH₂ₙY, -CH₂CHR₁CₙH₂ₙY, 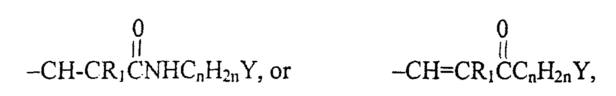 wherein R₁ is hydrogen or C₁₋₆ lower alkyl, n is 0 to 20, and Y is amino, carboxy, hydroxy, carboxyphenyl, aminoalkylphenyl or an adduct thereof, and said reporter group is bound to Y.

22. The process of any one of claims 15 to 20 wherein said modified base is adenine and said substituent group is CHR₁CₙH₂ₙY wherein R₁ is hydrogen or C₁₋₆ lower alkyl, n is 0 to 20, and Y is amino, carboxy, hydroxy, carboxyphenyl, aminoalkylphenyl or an adduct thereof, said substituent being attached to the C-8 position of said base through a nitrogen, oxygen or sulfur atom, and prior to or after said coupling step, there is bound to said substituent group a functionally colorimetric, luminescent, fluorescent or ligand recognition type non-radioisotopic reporter group, said non-radioisotopic reporter group, when bound prior to the coupling step, being in masked form if it would otherwise adversely affect the coupling reaction.

23. The process of claim 21 or claim 22 wherein said non-radioisotopic reporter group is bound to said substituent group prior to the coupling step and is nitrophenyl, dinitrophenyl, benzenesulfonyl, desthiobiotin or an adduct thereof.

24. The process of any one of claims 15 to 20 wherein said modified base is uracil or cytosine, and said substituent group has no non-radioisotopic reporter groups bound thereto and is -CH=CR₁CₙH₂ₙY, -CH₂CHR₁CₙH₂ₙY, 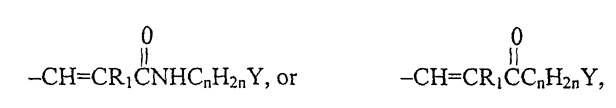 wherein R₁ is hydrogen or C₁₋₆ lower alkyl, n is 0 to 20, and Y is at least one masked amine, masked carboxy, masked hydroxy, masked carboxyphenyl, masked aminoalkylphenyl or an adduct thereof.

25. The process of any one of claims 15 to 20 wherein said modified base is adenine and said substituent group has no non-radioisotopic reporter group bound thereto and is -CHR₁CₙH₂ₙY wherein R₁ is hydrogen or C₁₋₆ lower alkyl, n is 0 to 20, and Y is at least one masked amine, masked carboxy, masked hydroxy, masked carboxyphenyl, masked aminoalkylphenyl or an adduct thereof, said substituent group being attached to the C-8 position of said base through a nitrogen, oxygen or sulfur atom.

26. The process of claim 24 or claim 25 wherein Y is at least one 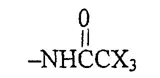 in which X is hydrogen, fluorine or chlorine.

27. The process of any one of claims 24 to 26 wherein, after the coupling step, Y is unmasked, and a solid support or at least one functionally colorimetric, luminescent, fluorescent, or ligand recognition type non-radioisotopic reporter group is bound to Y.

28. The process of claim 27 wherein said non-radioisotopic reporter group is fluorescein, fluorescamine, rhodamine, an acridinium salt, biotin, iminobiotin, desthiobiotin, luminol, isoluminol, luciferin, dioxetane, dioxamide, nitrophenyl, dinitrophenyl, benzenesulfonyl or an adduct thereof.

29. The process of any one of claims 15 to 28 wherein the nucleotide monomer becomes a new terminal unit of said growing nucleotide chain upon coupling thereto, and after the coupling step, the nucleotide chain is extended by sequentially coupling one or more additional nucleotide monomers to the newly formed terminal unit thereof.

30. The process of claim 29 wherein chain extension is continued by sequential coupling of additional nucleotide monomers to the nucleotide chain until an oligonucleotide as defined in any one of claims 1 to 13 is produced.

31. A compound useful as an intermediate in the chemical synthesis of an oligonucleotide and having the structure: 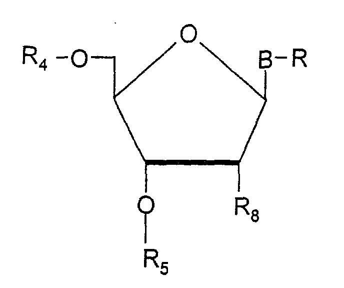 wherein
B is a pyrimidine base,
R is -CH=CR₁CₙH₂ₙY, -CH₂CHR₁CₙH₂ₙY, 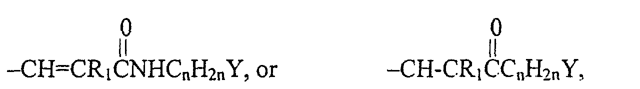 wherein R₁ is hydrogen or C₁₋₆ lower alkyl, n is 0 to 20, and Y is at least one masked amine or masked aminoalkylphenyl or is at least one amine or aminoalkylphenyl to which a reporter group is attached, which reporter group comprises nitrophenyl, dinitrophenyl, benzenesulfonyl, desthiobiotin or an adduct thereof, R being attached to said base at the C-5 position,
R₄ is a masking group or 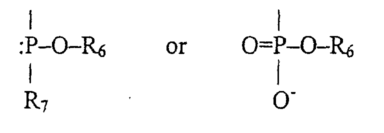
R₅ is 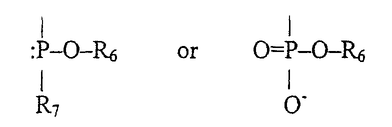
when R₄ is a masking group, and is a masking group when R₄ is 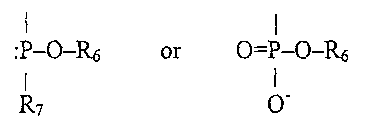 wherein R₆ is methyl or chlorophenyl, and R₇ is chloro, dialkyiamino or morpholino, R₈ is hydrogen or masked hydroxy, and said masking group is monomethoxytrityl, dimethoxytrityl, or trimethoxytrityl.

32. The compound of claim 31 wherein B is uracil.

33. The compound of claims 31 or 32 wherein n is 3 to 12.

34. The compound of any one of claims 31 to 33 wherein Y is at least one 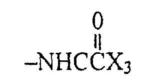 in which X is hydrogen, fluorine or chlorine.

35. A compound useful as an intermediate in the chemical synthesis of an oligonucleotide having the structure: 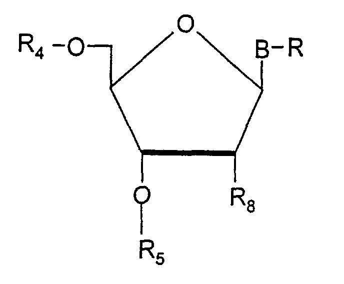 wherein
B is an adenine base,
R is a chain of one or more carbon atoms and at least a masked amine is attached to one end of said carbon chain, the other end of said carbon chain being attached to said base at the C-8 position through a polyvalent heteroatom,
R₄ is a masking group or 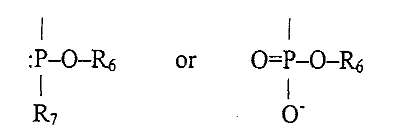
R₅ is 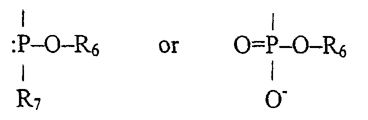 when R₄ is a masking group, and is a masking group when R₄ is 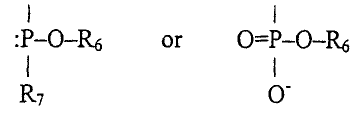
wherein R₆ is methyl or chlorophenyl, and R₇ is chloro, dialkylamino or morpholino, R₈ is hydrogen or masked hydroxy, and said masking group is monomethoxytrityl, dimethoxytrityl, or trimethoxytrityl.

36. The compound of claim 35 wherein R is attached to said base through a nitrogen atom.

37. The compound of claim 35 or claim 36 wherein R is -CHR₁CₙH₂ₙY, in which R₁ is hydrogen or C₁₋₆ lower alkyl, n is 6 and Y is at least one 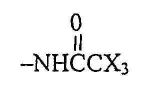 in which X is hydrogen, fluorine or chlorine.

## Patentansprüche

1. Homogenes Einzelstrang-Oligonukleotid definierter Sequenz mit weniger als 0,2 kb Länge, welches mindestens eine Ribonukleotid- oder Desoxyribonukleotid-Einheit enthält, die eine Pyrimidin- oder Purin-Base aufweist, welche an einer sterisch zulässigen Stelle modifiziert ist durch Anbringung einer Substituentengruppe daran, die mindestens eine nicht-radioisotopische Markergruppe oder einen festen Träger gebunden hat, wobei die Substituentengruppe, sofern die modifizierte Base ein Pyrimidin ist, eine Kette von zwei oder mehr Kohlenstoffatomen enthält, und sofern die modifizierte Base ein Purin ist, eine Kette von einem oder mehreren Kohlenstoffatomen enthält, die durch ein polyvalentes Heteroatom an die Base gebunden ist, wobei jede der so modifizierten Basen sich an einer vorher ausgewählten Stelle in dem Oligonukleotid befindet.

2. Oligonukleotid nach Anspruch 1, worin der Substituent an der C-5-Position einer Pyrimidin-Base oder an der C-8-Position einer Purin-Base gebunden ist.

3. Oligonukleotid nach Anspruch 1 oder Anspruch 2, worin die Substitutentengruppe an einen festen Träger gebunden ist oder zumindest eine nicht-radioisotopische Markergruppe gebunden hat, die funktional kolorimetrisch, fluoreszierend, lumineszierend oder vom Liganden-Erkennungs-Typ ist.

4. Oligonukleotid nach einem der voranstehenden Ansprüche, worin die modifizierte Base ein Pyrimidin ist, und die Markergruppe oder der feste Träger durch die Substitutentengruppe gebunden wurde, die mindestens eine endständige Amingruppe aufweist, welche an der Kohlenstoffkette gebunden ist, die von der C-5-Position entfernt ist.

5. Oligonukleotid nach Anspruch 4, worin ein fester Träger oder eine nicht-radioisotopische Markergruppe, die funktional kolorimetrisch, fluoreszierend, lumineszierend oder vom Liganden-Erkennungs-Typ ist, an die Amingruppe gebunden wurde.

6. Oligonukleotid nach einem der Ansprüche 1 bis 3, worin die modifizierte Base Uracil oder Cytosin ist und die Substituentengruppe
-CH=CR₁CₙH₂ₙY, -CH₂CHR₁CₙH₂ₙY, 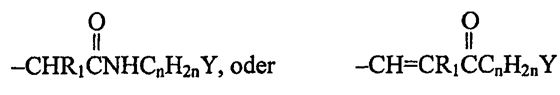 war, worin R₁ ein Wasserstoffatom oder ein C₁₋₆-Niederalkylrest ist, n eine Zahl von 0-20 ist, und Y mindestens eine Aminogruppe oder eine maskierte Aminogruppe, eine Carboxylgruppe oder maskierte Carboxylgruppe, ein Hydroxylrest oder ein maskierter Hydroxylrest, ein Carboxyphenylrest oder ein maskierter Carboxyphenylrest, ein Aminoalkylphenylrest oder ein maskierter Aminoalkylphenylrest oder ein Addukt davon ist.

7. Oligonukleotid nach einem der Ansprüche 1 bis 3, worin die modifizierte Base Adenin ist und die Substituentengruppe -CHR₁CₙH₂ₙY war und an die C-8-Position der Adenin-Base über ein Stickstoffatom, Sauerstoffatom oder Schwefelatom gebunden wurde, R₁ ein Wasserstoffatom oder ein C₁₋₆-Niederalkylrest ist, n 0 bis 20 ist, und Y mindestens eine Aminogruppe oder eine maskierte Aminogruppe, ein Hydroxylrest oder ein maskierter Hydroxylrest, eine Carboxylgruppe oder maskierte Carboxylgruppe, ein Carboxyphenylrest oder ein maskierter Carboxyphenylrest, ein Aminoalkylphenylrest oder ein maskierter Aminoalkylphenylrest oder ein Addukt davon ist.

8. Oligonukleotid nach Anspruch 6 oder Anspruch 7, worin n 3 bis 12 ist.

9. Oligonukleotid nach einem der Ansprüche 6 bis 8, worin mindestens eine nicht-radioisotopische Markergruppe, die funktional kolorimetrisch, fluoreszierend, lumineszierend oder vom Liganden-Erkennungs-Typ ist, an Y gebunden ist.

10. Oligonukleotid nach einem der voranstehenden Ansprüche, worin die mindestens eine nicht-radioisotopische Markergruppe Fluorescein, Fluorescamin, Rhodamin, ein Acridiniumsalz, Nitrophenyl, Dinitrophenyl, Benzolsulfonyl, Biotin, Iminobiotin, Desthiobiotin, Luminol, Isoluminol, Luciferin, Dioxetan, Dioxamid oder ein Addukt davon ist.

11. Oligonukleotid nach Anspruch 10, worin die Substituentengruppe über ein Stickstoffatom an die Adenin-Base gebunden ist und mindestens eine Markergruppe an Y gebunden ist, wobei die Markergruppe Fluorescein, Fluorescamin, Rhodamin, ein Acridiniumsalz, Nitrophenyl, Dintitrophenyl, Benzolsulfonyl, Luminol, Isoluminol, Luciferin, Dioxetan, Dioxamid, Biotin, Iminobiotin, Desthiobiotin oder ein Addukt davon ist.

12. Oligonukleotid nach einem der Ansprüche 6 bis 8, welches an einen festen Träger über Y gebunden wurde.

13. Oligonukleotid nach einem der voranstehenden Ansprüche, welches eine Länge von etwa 5 bis etwa 60 Baseneinheiten aufweist.

14. Oligonukleotid nach Anspruch 13, welches ein Länge von etwa 10 bis etwa 40 Baseneinheiten aufweist.

15. Verfahren zur chemischen Synthese eines Einzelstrang-Oligonukleotids definierter Sequenz, welches mindestens eine modifizierte Base aufweist, umfassend das Kondensieren eines Nukleotid-Monomers, welches gebunden eine aktivierte phosphorhaltige Gruppe aufweist, an eine Endgruppe, die einen ungeschützten Hydroxylrest trägt, einer Nukleotidkette an einer vorher ausgewählten Stelle, wodurch die Kette verlängert wird durch Kopplung des Monomers an sie über die aktive phosphorhaltige Gruppe an der Stelle des ungeschützten Hydroxyls, wobei die Nukleotidkette weniger als 0,2 kb aufweist, und mindestens eine der Monomer- und Endgruppeneinheiten eine Pyrimidin- oder Purin-Base eines Ribonukleotids oder Desoxyribonukleotids aufweist, welche an einer sterisch zulässigen Stelle modifiziert ist durch Anfügung einer Substituentengruppe, wobei die Substituentengrupe, sofern die modifizierte Base ein Pyrimidin ist, eine Kette von zwei oder mehr Kohlenstoffatomen aufweist, und sofern die modifizierte Base ein Purin ist, eine Kette von einem oder mehr Kohlenstoffatomen aufweist, die durch ein polyvalentes Heteroatom an die Base gebunden sind, wobei vor oder nach dem Kopplungsschritt mindestens eine nicht-radioisotopische Markergruppe oder ein fester Träger an die Substituentengruppe gebunden ist, wobei die Substituentengruppe während des Kopplungsschrittes keine daran gebundene Markergruppe daran gebunden aufweist oder eine maskierte oder unmaskierte Markergruppe daran gebunden aufweist, welche keinen wesentlichen gegenteiligen Effekt auf die Kondensierungsreaktion besitzt.

16. Verfahren nach Anspruch 15, worin die Ribonukleotid- oder Desoxyribonukleotid-Base an der C-5-Position einer Pyrimidin-Base und an der C-8-Position einer Purin-Base modifiziert ist.

17. Verfahren nach Anspruch 15 oder Anspruch 16, worin die phosphorhaltige Gruppe sich an der 3'- oder 5'-Position des Nukleotidmonomers befindet und die ungeschützte Hydroxylgruppe der endständigen Einheit sich an der 5'-Position der letzteren befindet, wenn die phosphorhaltige Gruppe an der 3'-Position des Monomers ist, und befindet sich an der 3'-Position der endständigen Einheit, wenn die phosphorhaltige Gruppe an der 5'-Position des Monomers ist.

18. Verfahren nach einem der Ansprüche 15 bis 17, worin vor oder nach dem Kopplungsschritt mindestens das Monomer oder die Nukleotidkette an einen festen Träger gebunden ist.

19. Verfahren nach einem der Ansprüche 15 bis 17, worin vor oder nach dem Kopplungsschritt mindestens eine nicht-radioisotopische Markergruppe, die funktional kolorimetrisch, fluoreszierend, lumineszierend oder vom Liganden-Erkennungs-Typ ist, an die Substitutentengruppe gebunden ist, wobei die Markergruppe, wenn sie vor dem Kopplungsschritt gebunden wird, in maskierter Form vorliegt, sofern sie in anderer Form einen ungünstigen Effekt auf die Kopplungsreaktion ausüben würde.

20. Verfahren nach Anspruch 19, worin nach dem Kopplungsschritt mindestens eine nicht-radioisotopischeMarkergruppe an die Substituentengruppe gebunden ist und es sich dabei um Fluorescein, Fluorescamin, Rhodamin, ein Acridiniumsalz, Nitrophenyl, Dinitrophenyl, Benzolsulfonyl, Biotin, Iminobiotin, Desthiobiotin, Luminol, Isoluminol, Luciferin, Dioxetan, Dioxamid oder ein Addukt davon handelt.

21. Verfahren nach einem der Ansprüche 15 bis 20, worin die modifizierte Base Uracil oder Cytosin ist und die Substituentengruppe
-CH=CR₁CₙH₂ₙY, -CH₂CHR₁CₙH₂ₙY, 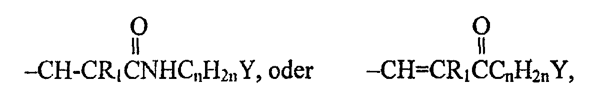 ist, worin R₁ ein Wasserstoffatom oder ein C₁₋₆-Niederalkylrest ist, n eine Zahl von 0 bis 20 ist und Y Amino, Carboxy, Hydroxy, Carboxyphenyl oder ein Addukt davon ist und die Markergruppe an Y gebunden ist.

22. Verfahren nach einem der Ansprüche 15 bis 20, worin die modifizierte Base Adenin ist und die Substituentengruppe -CHR₁CₙH₂ₙY ist, worin R₁ ein Wasserstoffatom oder ein C₁₋₆-Niederalkylrest ist, n 0 bis 20 ist und Y Amino, Carboxyl, Hydroxyl, Carboxyphenyl, Aminoalkylphenyl oder ein Addukt davon ist, wobei der Substituent an die C-8-Position der Base über ein Stickstoff-, Sauerstoff- oder Schwefelatom gebunden ist, und vor oder nach dem Kopplungsschritt an die Substituentengruppe eine nicht-radioisotopische Markergruppe, die funktional kolorimetrisch, fluoreszierend, lumineszierend oder vom Liganden-Erkennungs-Typ ist, gebunden wird, wobei die nicht-radioisotopische Markergruppe, sofern sie vor dem Kopplungsschritt gebunden wird, in maskierter Form vorliegt, sofern sie in anderer Weise die Kopplungsreaktion beeinträchtigen würde.

23. Verfahren nach Anspruch 21 oder Anspruch 22, worin die nicht-radioisotopische Markergruppe an die Substituentengruppe vor dem Kopplungsschritt gebunden wird und es sich um Nitrophenyl, Dinitrophenyl, Benzolsufonyl, Desthiobiotin oder ein Addukt davon handelt.

24. Verfahren nach einem der Ansprüche 15 bis 20, worin die modifizierte Base Uracil oder Cytosin ist, und die Substituentengruppe keine daran gebundene nicht-radioisotopische Markergruppe aufweist und
-CH=CR₁CₙH₂ₙY, -CH₂CHR₁CₙH₂ₙY, 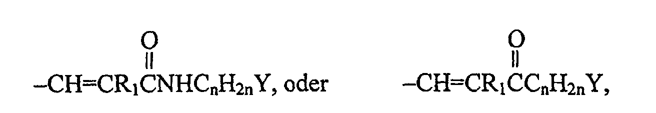 ist, worin R₁ ein Wasserstoffatom oder eine C₁₋₆-Niederalkylgruppe ist, n 0 bis 20 ist, und Y mindestens eine maskierte Aminogruppe, eine maskierte Carboxylgruppe, ein maskierter Hydroxylrest, ein maskierter Carboxyphenylrest, ein maskierter Aminoalkylphenylrest oder ein Addukt davon ist.

25. Verfahren nach einem der Ansprüche 15 bis 20, worin die modifizierte Base Adenin ist und die Substituentengruppe keine daran gebundene nicht-radioisotopische Markergruppe aufweist und für -CHR₁CₙH₂ₙY steht, worin R₁ ein Wasserstoffatom oder ein C₁₋₆-Niederalkylrest ist, n 0 bis 20 ist, und Y mindestens eine maskierte Aminogruppe, eine maskierte Carboxylgruppe, ein maskierter Hydroxylrest, ein maskierter Carboxyphenylrest, ein maskierter Aminoalkylphenylrest, oder ein Addukt davon ist, wobei die Substituentengruppe an die C-8-Position der Base über ein Stickstoff-, Sauerstoff- oder Schwefelatom gebunden ist.

26. Verfahren nach Anspruch 24 oder Anspruch 25, worin Y mindestens ein Rest 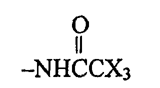 ist, worin X für ein Wasserstoff-, Fluor- oder Chloratom steht.

27. Verfahren nach einem der Ansprüche 24 bis 26, worin nach dem Kopplungsschritt Y unmaskiert ist und ein fester Träger oder mindestens eine nicht-radioisotopische Markergruppe, die funktional kolorimetrisch, fluoreszierend, lumineszierend oder vom Liganden-Erkennungs-Typ ist, an Y gebunden ist.

28. Verfahren nach Anspruch 27, worin die nicht-radioisotopische Markergruppe Fluorescein, Fluorescamin, Rhodamin, ein Acridiniumsalz, Biotin, Iminobiotin, Desthiobiotin, Luminol, Isoluminol, Luciferin, Dioxetan, Dioxamid, Nitrophenyl, Dinitrophenyl, Benzolsulfonyl oder ein Addukt davon ist.

29. Verfahren nach einem der Ansprüche 15 bis 28, worin das Nukleotidmonomer eine neue Endgruppeneinheit der wachsenden Nukleotidkette durch die Kopplung daran wird und nach dem Kopplungsschritt die Nukleotidkette verlängert wird durch aufeinanderfolgende Kopplung von einem oder mehreren zusätzlichen Nukleotidmonomeren zu der neu gebildeten endständigen Einheit davon.

30. Verfahren nach Anspruch 29, worin die Kettenverlängerung fortgeführt wird durch aufeinanderfolgende Kopplung von zusätzlichen Nukleotidmonomeren an die Nukleotidkette, bis ein Oligonukleotid erzeugt ist gemäß einem der Ansprüche 1 bis 13.

31. Eine als Zwischenverbindung bei der chemischen Synthese eines Oligonukleotids geeignete Verbindung mit der Struktur 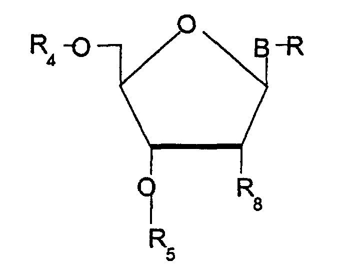 worin B eine Pyrimidin-Base ist, R für
-CH=CR₁CₙH₂ₙY, -CH₂CHR₁CₙH₂ₙY, 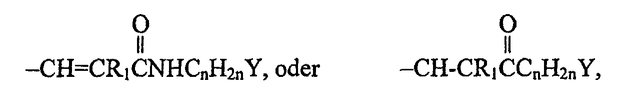 steht, worin R₁ ein Wasserstoffatom oder ein C₁₋₆-Niederalkylrest ist, n 0 bis 20 ist und Y mindestens ein maskiertes Amin oder ein maskierter Aminoalkylphenylrest ist oder mindestens ein Amin oder ein Aminoalkylphenylrest ist, woran eine Markergruppe gebunden ist, wobei die Markergruppe Nitrophenyl, Dinitrophenyl, Benzolsulfonyl, Desthiobiotin oder ein Addukt davon umfasst, wobei R an die Base an der C-5-Position gebunden ist,
R₄ eine Maskierungsgruppe oder 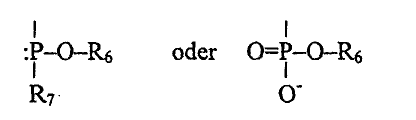 ist, R₅ für 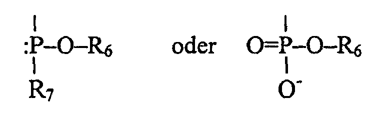 steht, wenn R₄ eine Maskierungsgruppe ist, und eine Maskierungsgruppe ist, wenn R₄ 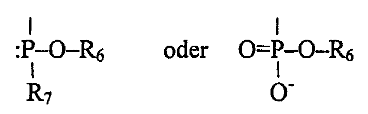 ist, worin R₆ ein Methylrest oder ein Chlorphenylrest ist, und R₇ ein Chloratom, eine Dialkylaminogruppe oder eine Morpholinogruppe bedeutet,
R₈ ein Wasserstoffatom oder eine maskierte Hydroxylgruppe darstellt, und die Maskierungsgruppe Monomethoxytrityl, Dimethoxytrityl oder Trimethoxytrityl ist.

32. Verbindung nach Anspruch 31, worin B Uracil ist.

33. Verbindung nach einem der Ansprüche 31 oder 32, worin n 3 bis 12 ist.

34. Verbindung nach einem der Ansprüche 31 bis 33, worin Y mindestens ein 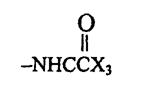 ist, worin X für ein Wasserstoff-, Fluor- oder Chloratom steht.

35. Eine als Zwischenverbindung bei der chemischen Synthese eines Oligonukleotids geeignete Verbindung mit der Struktur 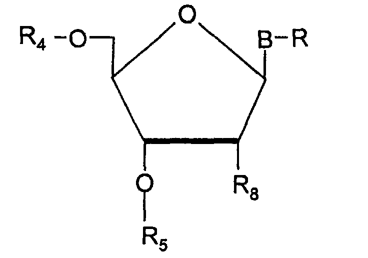 worin B eine Adenin-Base ist,
R eine Kette von einem oder mehreren Kohlenstoffatome darstellt und mindestens eine maskierte Aminogruppe an ein Ende der Kohlenstoffkette gebunden ist, wobei das andere Ende der Kohlenstoffkette über ein polyvalentes Heteroatom an die Base an der C-8-Position gebunden ist,
R₄ eine Maskierungsgruppe oder 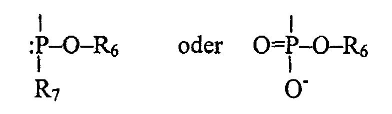 bedeutet,
R₅ für 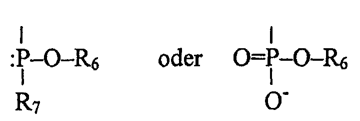 steht, wenn R₄ eine Maskierungsgruppe ist, und eine Maskierungsgruppe bedeutet, wenn R₄ 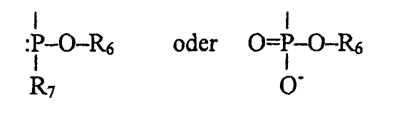 ist, worin R₆ ein Methylrest oder ein Chlorphenylrest ist, und R₇ ein Chloratom, eine Dialkylaminogruppe oder ein Morpholinorest ist,
R₈ ein Wasserstoffatom oder einen maskierten Hydroxylrest bedeutet, und die Maskierungsgruppe Monomethoxytrityl, Dimethoxytrityl oder Trimethoxytrityl ist.

36. Verbindung nach Anspruch 35, worin R über ein Stickstoffatom an die Base gebunden ist.

37. Verbindung nach Anspruch 35 oder Anspruch 36, worin R für -CHR₁CₙH₂ₙY steht, worin R₁ ein Wasserstoffatom oder ein C₁₋₆-Niederalkylrest ist, n gleich 6 ist, und Y mindestens ein Rest 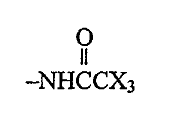 ist, worin X für ein Wasserstoff-, Fluor- oder Chloratom steht.

## Revendications

1. Oligonucléotide simple-brin à séquence homogène définie, ayant une longueur de moins de 0,2 kb comportant au moins une unité ribonucléotidique ou désoxyribonucléotidique ayant une base pyrimidine ou purine modifiée au niveau d'un site stériquement tolérant, par fixation à celui-ci d'un groupe substituant qui a au moins un groupe reporteur non radio-isotope ou un support solide lié à celui-ci, le groupe substituant, lorsque la base modifiée est une pyrimidine, comportant une chaîne de deux ou plus de deux atomes de carbone, et lorsque la base modifiée est une purine, comportant une chaîne d'un ou plusieurs atomes de carbone fixés à la base par l'intermédiaire d'un hétéro-atome polyvalent, chaque base modifiée étant située à une position présélectionnée dans l'oligonucléotide.

2. Oligonucléotide selon la revendication 1, dans lequel le substituant est fixé à la position C-5 d'une base pyrimidine ou à la position C-8 d'une base purine.

3. Oligonucléotide selon la revendication 1 ou 2, dans lequel le groupe substituant est lié à un support solide ou a lié à celui-ci au moins un groupe reporteur non radio-isotope fonctionnellement colorimétrique, fluorescent, luminescent ou du type à reconnaissance de ligand.

4. Oligonucléotide selon l'une quelconque des revendications précédentes, dans lequel ladite base modifiée est une pyrimidine, et le groupe reporteur ou support solide a été lié par le groupe substituant ayant au moins un groupe terminal amine fixé à ladite chaîne carbonée à distance de la position C-5.

5. Oligonucléotide selon la revendication 4, dans lequel un support solide ou un groupe reporteur non radio-isotope fonctionnellement colorimétrique, fluorescent, luminescent ou du type à reconnaissance de ligand a été fixé audit groupe amine.

6. Oligonucléotide selon l'une quelconque des revendications 1 à 3, dans lequel ladite base modifiée est un uracile ou une cytosine et ledit groupe substituant est -CH=CR₁CₙH₂ₙY, -CH₂CHR₁CₙH₂ₙY, 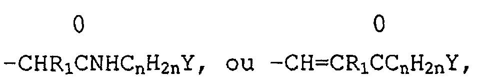 où R₁ est l'hydrogène ou un groupe alkyle inférieur en C₁₋₆, n est compris entre 0 et 20, et Y est au moins une amine ou une amine masquée, un groupe carboxy ou carboxy masqué, un groupe hydroxy ou hydroxy masqué, un groupe carboxyphényle ou carboxyphényle masqué, un groupe aminoalkylphényle ou aminoalkylphényle masqué, ou un produit d'addition de ceux-ci.

7. Oligonucléotide selon l'une quelconque des revendications 1 à 3, dans lequel ladite base modifiée est une adénine et ledit groupe substituant était -CH₁CₙH₂ₙY et a été fixé à la position C-8 de ladite base adénine par l'intermédiaire d'un atome d'azote, d'oxygène ou de soufre, R₁ est l'hydrogène ou un groupe alkyle inférieur en C₁₋₆, n est compris entre 0 et 20, et Y est au moins une amine ou une amine masquée, un groupe hydroxy ou hydroxy masqué, un groupe carboxy ou carboxy masqué, un groupe carboxyphényle ou carboxyphényle masqué, un groupe aminoalkylphényle ou aminoalkylphényle masqué, ou un produit d'addition de ceux-ci.

8. Oligonucléotide selon la revendication 6 ou 7, dans lequel n est compris entre 3 et 12.

9. Oligonucléotide selon l'une quelconque des revendications 6 à 8, dans lequel au moins un groupe reporteur non radio-isotope fonctionnellement colorimétrique, luminescent ou fluorescent ou du type à reconnaissance de ligand est lié à Y.

10. Oligonucléotide selon l'une quelconque des revendications précédentes, dans lequel ledit groupe reporteur non radio-isotope, au nombre d'au moins un, est une fluorescéine, une fluorescamine, une rhodamine, un sel d'acridinium, un nitrophényle, un dinitrophényle, un benzènesulfonyle, une biotine, une iminobiotine, une desthiobiotine, un luminol, un isoluminol, une luciférine, un dioxétane, un dioxamide ou un produit d'addition de ceux-ci.

11. Oligonucléotide selon la revendication 10, dans lequel le groupe substituant est fixé à la base adénine au moyen d'un atome d'azote, et au moins un groupe reporteur est lié à Y, lequel groupe reporteur est une fluorescéine, une fluorescamine, une rhodamine, un sel d'acridinium, un nitrophényle, un dinitrophényle, un benzènesulfonyle, un luminol, un isoluminol, une luciférine, un dioxétane, un dioxamide, une biotine, une iminobiotine, une desthiobiotine ou un produit d'addition de ceux-ci.

12. Oligonucléotide selon l'une quelconque des revendications 6 à 8, qui a été fixé à un support solide par Y.

13. Oligonucléotide selon l'une quelconque des revendications précédentes, qui a une longueur d'environ 5 à environ 60 unités de bases.

14. Oligonucléotide selon la revendication 13, qui a une longueur d'environ 10 à environ 40 unités de bases.

15. Procédé de synthèse chimique d'un oligonucléotide simple-brin à séquence définie comportant au moins une base modifiée, comprenant la condensation en une unité terminale portant un groupe hydroxyle non protégé d'une chaîne nucléotidique à une position présélectionnée, un monomère nucléotidique ayant fixé à celui-ci un groupe contenant du phosphore activé, de sorte que ladite chaîne est étendue par couplage dudit monomère à celle-ci par l'intermédiaire dudit groupe contenant du phosphore actif au niveau du site dudit groupe hydroxyle non protégé, ladite chaîne nucléotidique ayant moins de 0,2 kb, et au moins un élément parmi ledit monomère et lesdites unités terminales ayant une base pyrimidine ou purine ribonucléotidique ou désoxyribonucléotidique modifiée au niveau d'un site stériquement tolérant, par fixation à celui-ci d'un substituant, le groupe substituant, lorsque la base modifiée est une pyrimidine, comportant une chaîne de deux ou plus de deux atomes de carbone, et lorsque la base modifiée est une purine, comportant une chaîne d'un ou plusieurs atomes de carbone fixés à la base par l'intermédiaire d'un hétéro-atome polyvalent, dans lequel, avant ou après l'étape de couplage, au moins un groupe reporteur non radio-isotope ou support solide est lié audit substituant, ledit groupe substituant pendant ladite étape de couplage n'ayant aucun groupe reporteur lié à celui-ci ou ayant un groupe reporteur masqué ou non masqué lié à celui-ci, qui n'a pas d'effet néfaste sensible sur ladite réaction de condensation.

16. Procédé selon la revendication 15, dans lequel la base ribonucléotidique ou désoxyribonucléotidique est modifiée au niveau de la position C-5 d'une base pyrimidine et au niveau de la position C-8 d'une base purine.

17. Procédé selon la revendication 15 ou 16, dans lequel ledit groupe contenant du phosphore est à la position 3' ou 5' du monomère nucléotidique et ledit groupe hydroxyle non protégé de l'unité terminale est à la position 5' de cette dernière lorsque le groupe contenant du phosphore est à la position 3' du monomère et est à la position 3' de l'unité terminale lorsque le groupe contenant du phosphore est à la position 5' du monomère.

18. Procédé selon l'une quelconque des revendications 15 à 17, dans lequel, avant ou après l'étape de couplage, au moins un élément choisi parmi ledit monomère et ladite chaîne nucléotidique est fixé à un support solide.

19. Procédé selon l'une quelconque des revendications 15 à 17, dans lequel, avant ou après l'étape de couplage, au moins un groupe reporteur non radio-isotope fonctionnellement colorimétrique, fluorescent, luminescent ou du type à reconnaissance de ligand est lié audit groupe substituant, ledit groupe reporteur, lorsqu'il est lié avant l'étape de couplage, étant sous une forme masquée, sinon il pourrait affecter de manière néfaste la réaction de couplage.

20. Procédé selon la revendication 19, dans lequel ledit groupe reporteur non radio-isotope, au nombre d'au moins un, est lié audit groupe substituant après l'étape de couplage et est une fluorescéine, une fluorescamine, une rhodamine, un sel d'acridinium, un nitrophényle, un dinitrophényle, un benzènesulfonyle, une biotine, une iminobiotine, une desthiobiotine, un luminol, un isoluminol, une luciférine, un dioxétane, un dioxamide ou un produit d'addition de ceux-ci.

21. Procédé selon l'une quelconque des revendications 15 à 20, dans lequel ladite base modifiée est un uracile ou une cytosine et ledit groupe substituant est -CH=CR₁CₙH₂ₙY, -CH₂CHR₁CₙH₂ₙY, 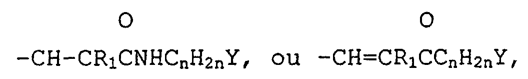 où R₁ est l'hydrogène ou un groupe alkyle inférieur en C₁₋₆, n est compris entre 0 et 20, et Y est un groupe amino, carboxy, hydroxy, carboxyphényle, aminoalkylphényle ou un produit d'addition de ceux-ci, et ledit groupe reporteur est lié à Y.

22. Procédé selon l'une quelconque des revendications 15 à 20, dans lequel ladite base modifiée est une adénine et ledit groupe substituant est CHR₁CₙH₂ₙY où R₁ est l'hydrogène ou un groupe alkyle inférieur en C₁₋₆, n est compris entre 0 et 20, et Y est un groupe amino, carboxy, hydroxy, carboxyphényle, aminoalkylphényle ou un produit d'addition de ceux-ci, ledit substituant étant fixé à la position C-8 de ladite base par un atome d'azote, d'oxygène ou de soufre, et avant ou après ladite étape de couplage, on fixe audit groupe substituant un groupe reporteur non radio-isotope fonctionnellement colorimétrique, luminescent, fluorescent ou du type à reconnaissance de ligand, ledit groupe reporteur non radio-isotope, lorsqu'il est lié avant l'étape de couplage, étant sous une forme masquée car, sinon, il pourrait affecter de manière néfaste la réaction de couplage.

23. Procédé selon la revendication 21 ou 22, dans lequel ledit groupe reporteur non radio-isotope est lié audit groupe substituant avant l'étape de couplage et est un groupe nitrophényle, dinitrophényle, benzènesulfonyle, desthiobiotine ou un produit d'addition de ceux-ci.

24. Procédé selon l'une quelconque des revendications 15 à 20, dans lequel ladite base modifiée est un uracile ou une cytosine, et ledit groupe substituant n'a aucun groupe reporteur non radio-isotope lié à celui-ci et est -CH-CR₁CₙH₂ₙY, -CH₂CHR₁CₙH₂ₙY, 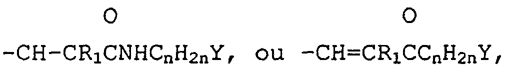 où R₁ est l'hydrogène ou un groupe alkyle inférieur en C₁₋₆, n est compris entre 0 et 20, et Y est au moins une amine masquée, un groupe carboxy masqué, un groupe hydroxy masqué, un groupe carboxyphényle masqué, un groupe aminoalkylphényle masqué ou un produit d'addition de ceux-ci.

25. Procédé selon l'une quelconque des revendications 15 à 20, dans lequel ladite base modifiée est une adénine et ledit groupe substituant n'a aucun groupe reporteur non radio-isotope lié à celui-ci et est -CHR₁CₙH₂ₙY où R₁ est l'hydrogène ou un groupe alkyle inférieur en C₁₋₆, n est compris entre 0 et 20, et Y est au moins une amine masquée, un groupe carboxy masqué, un groupe hydroxy masqué, un groupe carboxyphényle masqué, un groupe aminoalkylphényle masqué ou un produit d'addition de ceux-ci, ledit groupe substituant étant fixé à la position C-8 de ladite base par un atome d'azote, d'oxygène ou de soufre.

26. Procédé selon la revendication 24 ou 25, dans lequel Y est au moins un composé 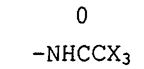 où X est l'hydrogène, le fluor ou le chlore.

27. Procédé selon l'une quelconque des revendications 24 à 26, dans lequel, après l'étape de couplage, Y est non masqué, et un support solide ou au moins un groupe reporteur non radio-isotope fonctionnellement colorimétrique, luminescent, fluorescent ou du type à reconnaissance de ligand est lié à Y.

28. Procédé selon la revendication 27, dans lequel ledit groupe reporteur non radio-isotope est une fluorescéine, une fluorescamine, une rhodamine, un sel d'acridinium, une biotine, une iminobiotine, une desthiobiotine, un luminol, un isoluminol, une luciférine, un dioxétane, un dioxamide, un nitrophényle, un dinitrophényle, un benzène-suifonyle ou un produit d'addition de ceux-ci.

29. Procédé selon l'une quelconque des revendications 15 à 28, dans lequel le monomère nucléotidique devient une nouvelle unité terminale de ladite chaîne nucléotidique en croissance lors du couplage à celle-ci, et après l'étape de couplage, la chaîne nucléotidique est étendue par couplage séquentiel d'un ou plusieurs monomères nucléotidiques supplémentaires à l'unité terminale nouvellement formée de celle-ci.

30. Procédé selon la revendication 29, dans lequel ladite extension de chaîne se poursuit par un couplage séquentiel de monomères nucléotidiques supplémentaires à la chaîne nucléotidique jusqu'à ce qu'un oligonucléotide tel que défini dans l'une quelconque des revendications 1 à 13 soit produit.

31. Composé pouvant être utilisé en tant que composé intermédiaire dans la synthèse chimique d'un oligonucléotide et ayant la structure : 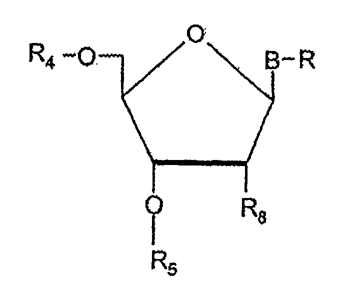 dans laquelle
B est une base pyrimidine,
R est -CH-CR₁CₙH₂ₙY, -CH₂CHR₁CₙH₂ₙY, 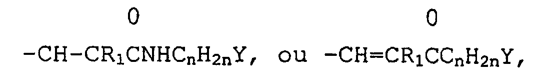 où R₁ est l'hydrogène ou un groupe alkyle inférieur en C₁₋₆, n est compris entre 0 et 20, et Y est au moins une amine masquée ou un groupe aminoalkylphényle masqué, ou est au moins une amine ou un groupe aminoalkylphényle auquel un groupe reporteur est fixé, lequel groupe reporteur comprend un groupe nitrophényle, dinitrophényle, benzènesulfonyle, desthiobiotine ou un produit d'addition de ceux-ci, R étant fixé à ladite base à la position C-5,
R₄ est un groupe de masquage ou 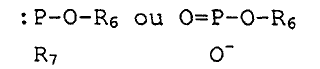
R₅ est 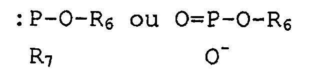 quand R₄ est un groupe de masquage, et est un groupe de masquage quand R₄ est 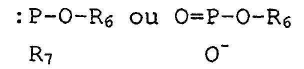 où R₆ est un groupe méthyle ou chlorophényle, et R₇ est un groupe chloro, dialkylamino ou morpholino, R₈ est l'hydrogène ou un groupe hydroxy masqué, et ledit groupe de masquage est un groupe monométhoxytrityle, diméthoxytrityle ou triméthoxytrityle.

32. Composé selon la revendication 31, dans lequel B est de l'uracile.

33. Composé selon la revendication 31 ou 32, dans lequel n est compris entre 3 et 12.

34. Composé selon l'une quelconque des revendications 31 à 33, dans lequel Y est au moins un composé 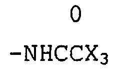 dans lequel X est l'hydrogène, le fluor ou le chlore.

35. Composé pouvant être utilisé en tant que composé intermédiaire dans la synthèse chimique d'un oligonucléotide ayant la structure : 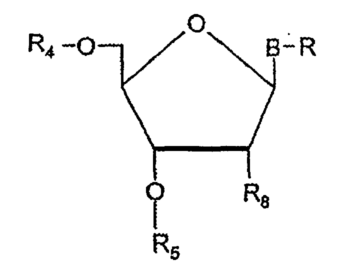 dans laquelle
B est une base adénine,
R est une chaîne d'un ou plusieurs atomes de carbone, et au moins une amine masquée est fixée à une extrémité de ladite chaîne carbonée, l'autre extrémité de ladite chaîne carbonée étant fixée à ladite base à la position C-8 par un hétéro-atome polyvalent,
R₄ est un groupe de masquage ou 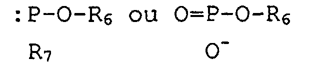
R₅ est 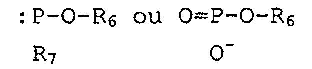 quand R₄ est un groupe de masquage, et est un groupe de masquage quand R₄ est 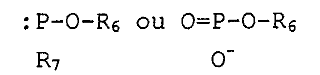 où R₆ est un groupe méthyle ou chlorophényle, et R₇ est un groupe chloro, dialkylamino ou morpholino, R₈ est l'hydrogène ou un groupe hydroxy masqué, et ledit groupe de masquage est un groupe monométhoxytrityle, diméthoxytrityle ou triméthoxytrityle.

36. Composé selon la revendication 35, dans lequel R est fixé à ladite base par un atome d'azote.

37. Composé selon la revendication 35 ou la revendication 36, dans lequel R est -CHR₁CₙH₂ₙY où R₁ est l'hydrogène ou un groupe alkyle inférieur en C₁₋₆, n vaut 6 et Y est au moins un composé 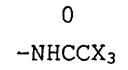 dans lequel X est l'hydrogène, le fluor ou le chlore.
